# FASCICULE DE BREVET EUROPEEN

(11) **EP 1 157 028 B1**
(45) Date de publication et mention de la délivrance du brevet: **04.09.2002**
(21) Numéro de dépôt: 00906440.3
(22) Date de dépôt: 21.02.2000
(51) Int. Cl.: C07J 41/00, A61K 31/565, A61K 31/58

(54) **NOUVEAUX DERIVES DE STEROIDES 11.BETA.-SUBSTITUES , LEURS PROCEDES DE PREPARATION ET INTERMEDIAIRES DE CE PROCEDE, LEUR APPLICATION A TITRE DE MEDICAMENTS ET COMPOSITIONS LES CONTENANT**
11.BETA.-SUBSTITUIERTE STEROIDE, VERFAHREN ZU IHRER HERSTELLUNG SOWIE ZWISCHENPRODUKTE DAVON, IHRE VERWENDUNG ALS MEDIKAMENTE UND PHARMAZEUTISCHE ZUSAMMENSETZUNGEN DAVON
NOVEL SUBSTITUTED 11.BETA STEROID DERIVATIVES, METHOD FOR PREPARING SAME AND INTERMEDIATES OF SAID METHOD, USE AS MEDICINE AND COMPOSITIONS CONTAINING THEM

(30) Priorité: 22.02.1999 FR 9902152
(43) Date de publication de la demande: 28.11.2001
(73) Titulaire: Aventis Pharma S.A., 92160 Antony (FR); Genentech, Inc., South San Francisco, CA 94080-4990 (US)
(72) Inventeur: CARNIATO, Denis, F-06800 Cagnes-sur-Mer (FR); GADEK, Thomas, R., Oakland, CA 94611 (US); GOURVEST, Jean-François, F-77140 Claye-Souilly (FR); KNOLLE, Jochen, D-65830 Kriftel (DE); PEYMAN, Anurschirwan, D-65779 Kelkheim (DE); BODARY, Sarah, C., San Bruno, CA 94066 (US)
(74) Mandataire: Rousseau, Pierrick Edouard
(86) Numéro de dépôt international: FR0000426
(87) Numéro de publication internationale: WO00050441

(56) Documents cités:
- EP-A- 0 384 842
- MARIONS, L. ET AL: "The effect of antiprogestin on integrin expression in human endometrium: an immunohistochemical study" MOL. HUM. REPROD. (1998), 4(5), 491-495, XP002118345
- CHEMICAL ABSTRACTS, vol. 114, no. 10, 11 mars 1991 (1991-03-11) Columbus, Ohio, US; abstract no. 88501, BASU, KRISHNAKALI ET AL: "Effects of 3-hydrazone modification on the metabolism and protein binding of progesterone" XP002118347 & INT. J. PHARM. (1990), 65(1-2), 109-14,
- CHEMICAL ABSTRACTS, vol. 92, no. 19, 12 mai 1980 (1980-05-12) Columbus, Ohio, US; abstract no. 164158, VOLOVEL'SKII, L. N. ET AL: "Synthesis and study of hydrazones of 17.alpha.- hydroxyprogesterone and.DELTA.4,6-pregnadien-17.alpha.-ol- 3,20-dione and their 17.alpha.-acylated derivatives" XP002118348 & KHIM. PRIR. SOEDIN. (1979), (4), 501-9,
- SHROFF, ARVIN P. ET AL: "Synthesis and biological response of some 3- iminoprogestins" J. MED. CHEM. (1971), 14(8), 769-70, XP002118346

## Description

La présente invention concerne de nouveaux dérivés de stéroïdes, leur procédé de préparation et les intermédiaires de ce procédé, leur application à titre de médicaments et les compositions pharmaceutiques les renfermant.

La présente invention a pour objet les composés de formule générale (I) : 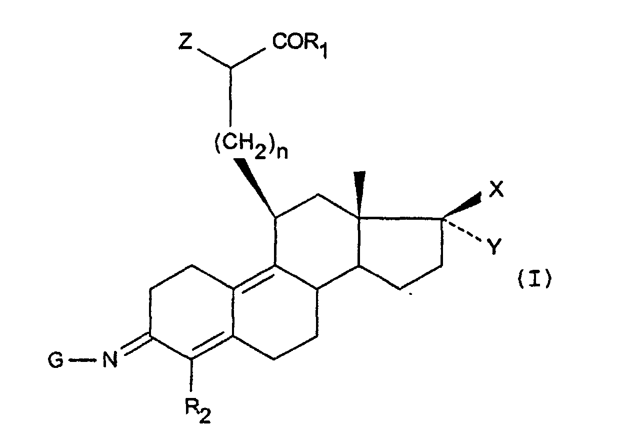 dans laquelle :
- soit X et Y forment ensemble avec le carbone qui les porte un groupement C=O ou C=CH₂,
- soit X représente un radical hydroxy, (C₁-C₆)-alkyloxy ou (C₁-C₆)-alkylcarbonyloxy, le reste alkyle étant non substitué ou substitué par R₃, et Y est un atome d'hydrogène,
- R₁ représente un radical hydroxy, (C₁-C₆)-alkyloxy, amino, (C₁-C₆)-alkylamino ou (C₂-C₁₂)-dialkylamino, le reste alkyle étant non substitué ou substitué par R₃,
- R₂ représente un atome d'hydrogène ou d'halogène,
- Z représente un atome d'hydrogène, un groupement MHSO₂Ra, NHCO₂Ra, NHCORa, NHSO₂NHRa ou NHCONHRa,
- G représente :
   - soit un radical 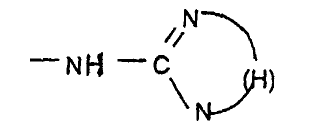 (radical G1) dans lequel (H) forme avec le motif -N=C-N- le reste d'un hétérocycle tel que défini ci-après,
   - soit un radical NRbRc (radical G2),
   - soit un hétérocycle (radical G3) tel que défini ci-après,
   - soit un radical NRb-C(=A)-NHRc (radical G4) dans lequel A est un atome de soufre, d'oxygène ou NH,
   - soit un radical NRb-SO₂Rc (radical G5),
- Ra, Rb et Rc, identiques ou différents, représentent un atome d'hydrogène, un radical -(CH₂)ₘ-Alk ou -(CH₂)ₘ-Ar
- le terme Alk représentant un radical dérivé d'un hydrocarbure, linéaire, ramifié ou cyclique, saturé ou insaturé, non aromatique renfermant de 1 à 12 atomes de carbone, substitué par R₃ ou non substitué, Ar représentant un phényle substitué par R₃ ou non substitué,
- n est un entier variant de 1 à 6, m représente 0,1,2 ou 3,
- le substituant R₃ représente :
   - halogène, oxo, cyano, nitro, formyl, carboxy, (C₁-C₆)-alkyloxycarbonyl, carboxamide,
   - un radical alkyle, alkényle ou alkynyle renfermant de 1 à 6 atomes de carbone éventuellement substitués par un ou plusieurs atomes d'halogène,
   - un radical cycloalkyle renfermant de 3 à 12 atomes de carbone,
   - un radical alkoxy ou alkylthio renfermant de 1 à 6 atomes de carbone,
   - un radical amino, alkylamino renfermant de 1 à 6 atomes de carbone ou dialkylamino renfermant de 2 à 12 atomes de carbone éventuellement sous forme oxydée,
   - un radical aminoalkyle renfermant de 1 à 6 atomes de carbone ou dialkylaminoalkyle renfermant de 3 à 8 atomes de carbone,
   - un radical dialkylaminoalkyloxy renfermant de 3 à 18 atomes de carbone,
   - un radical hydroxyle éventuellement acylé renfermant de 1 à 12 atomes de carbone,
   - un radical acyle renfermant de 1 à 12 atomes de carbone,
   - un radical phényle, phénylalkyle ou phényloxy, ces radicaux étant eux-mêmes éventuellement substitués par un ou plusieurs substituants cités plus haut,
lesdits composés de formules (I) étant sous toutes leurs formes stéréoisomères possibles, isolées ou en mélange, ainsi que leurs esters et leurs sels d'addition avec les acides et les bases pharmaceutiquement acceptables.

Par le terme Alk renfermant de 1 à 12 atomes de carbone, on désigne dans le cas des hydrocarbures acycliques les radicaux alkyles tels que méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthylheptyle ou n-décyle, les radicaux alkényles tels que vinyle, propényle, isopropényle, allyle, 2-méthylallyle, butényle ou isobutényle, ou les radicaux alkynyles tels que éthynyle, propynyle, propargyle, butylyle ou isobutynyle, et dans le cas des radicaux cycliques, les radicaux cycloalkyles, tels que cyclopropyle, cyclobutyle, cyclopentyle, cyclohexyle ou adamantyle. On entend de préférence les radicaux alkyles renfermant de 1 à 6 atomes de carbone et tout particulièrment méthyle, éthyle, propyle, isopropyle, butyle, isobutyle et tertbutyle.

Par hétérocycle (radical G₃), on entend un hétérocycle aromatique (hétéroaryle) ou non aromatique, saturé ou non saturé, comportant de 1 à 9 atomes de carbone et de 1 à 5 hétéroatomes choisis parmi les atomes d'oxygène, d'azote et de soufre, on désigne notamment :
- les radicaux monocycliques hétérocycliques, par exemple les radicaux thiényle, furyle, pyranyle, pyrrolyle, imidazolyle, pyrazolyle, pyridyle, pyrazinyle, pyrimidinyle, pyridazinyle, thiazolyle, oxazolyle, furazannyle, pyrrolinyle, imidazolinyle, pyrazolinyle, thiazolinyle, triazolyle, tétrazolyle,
- les cycles condensés hétérocycliques, par exemple le benzofurannyle, le benzothiényle, le benzymidazolyle, le benzothiazolyle, le naphto[2,3-b]thiényle, le thianthrényle, l'isobenzofurannyle, le chroményle, le xanthényle, le phénoxathiinyle, l'indolizinyle, l'isoindolyle, le 3H-indolyle, l'indolyle, l'indazolyle, le purinyle, le quinolizinyle, l'isoquinolyle, le quinolyle, le phtalazinyle, le naphtyridinyle, le quinoxalinyle, le quinazolinyle, le cinnolinyle, le ptéridinyle, le carbazolyle, le béta-carbolinyle, l'acridinyle, le phénazinyle, le phénothiazinyle, le phénoxazinyle, l'indolinyle, l'isoindolinyle, l'imidazopyridyle, l'imidazopyrimidinyle ou encore les systèmes polycycliques condensés constitués de monocycliques hétérocycliques tels que définis ci-dessus comme par exemple le furo[2,3-b]pyrrole ou le thiéno[2,3-b]furanne,
- ou les hétérocycles saturés tels que pyrrolidine, pipéridine ou morpholine.

Lorsque G est le radical G1, G1 représente un des hétérocycles suivants : 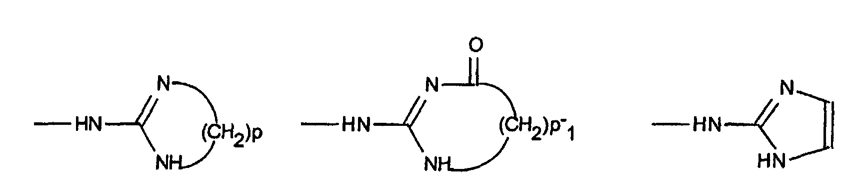 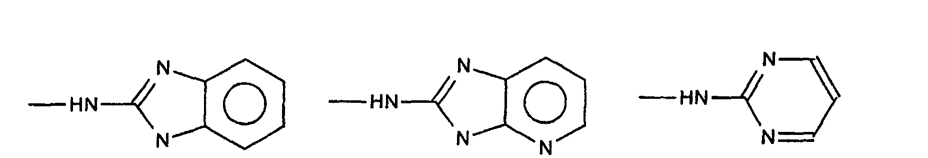 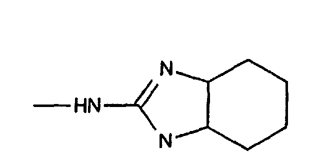 dans lesquels p représente un entier de 1 à 4 et de préférence 2 ou 3.

Lorsque G est le radical G2, G2 peut-être notamment un groupement amino, alkylamino tel que -NHMe, -NHEt, dialkylamino tel que -NMe₂, -NEt₂, -NMeEt, -NHPh, -NHCH₂Ph ou bien -NHCH₂-pyrrol-2-yle.

Lorsque G est le radical G4 ou G5, il s'agit notamment des groupements -NH-C(=NH)-NH₂, -NH-CO-NHCH₂Ph, -NHCONH₂, -NH-CS-NH₂, -NH-C(=NH)-NHCH₂Ph, - NH-C(=NH)-NHCH₃ ou -NHSO₂Ph.

Lorsque R₁ représente alkyloxy ou alkylamino il s'agit notamment des groupements suivants :
-OMe, -OEt, -O-(CH₂)₂-OH, -O-CH₂-CH(CH)-CH₂OH, -O-(CH₂)₂-NH₂, -O-(CH₂)₂-NMe₂ ou -OCH₂Ph.

Les substituants R₃ éventuels des groupements alkyles, phényles ou hétérocycles, tels que définis précédemment sont choisis parmi les radicaux suivants :
- halogène : fluor, chlore, brome, iode,
- alkyle, alkényle, alkynyle renfermant de 1 à 6 atomes de carbone tel que méthyle, éthyle, propyle, isopropyle, butyle, isobutyle, tert-butyle, vinyl ou allenyl. Ces radicaux étant eux-mêmes éventuellement substitués par un ou plusieurs atomes d'halogène, par exemple le fluor tel que le trifluorométhyle,
- cycloalkyle renfermant de 3 à 12 atomes de carbone tel que cyclohexyle ou adamantyle,
- oxo, cyano, nitro, formyl, carboxy et carboxyalkyl renfermant de 1 à 6 atomes de carbone, carboxamide,
- alkoxy renfermant de 1 à 6 atomes de carbone tel que méthoxy, éthoxy, propyloxy, isopropyloxy, butyloxy,
- alkylthio renfermant de 1 à 6 atomes de carbone tel que méthylthio, éthylthio, propylthio, isopropylthio, butylthio,
- amino, alkylamino renfermant de 1 à 6 atomes de carbone tel que méthylamino ou éthylamino, dialkylamino renfermant de 2 à 12 atomes de carbone tel que diméthylamino, diéthylamino, méthyléthylamino, chacun de ces radicaux dialkylamino étant éventuellement sous forme oxydée,
- aminoalkyle renfermant de 1 à 6 atomes de carbone tel que aminométhyle ou aminoéthyle,
- dialkylaminoalkyle renfermant de 3 à 18 atomes de carbone tel que diméthylamino méthyle ou éthyle,
- dialkylaminoalkyloxy renfermant de 3 à 18 atomes de carbone tel que diméthylaminoéthyloxy,
- hydroxyle éventuellement acylé renfermant de 1 à 12 atomes de carbone, par exemple acétoxy,
- acyle renfermant de 1 à 12 atomes de carbone tels que formyle, acétyle, propionyle, butyryle, isobutyryle, valéryle, isovaléryle, succinyle, pivaloyle, benzoyle,
- phényle, phénylalkyle tel que benzyle, phényloxy, ces radicaux étant eux-mêmes éventuellement substitués par les radicaux cités plus haut.

Bien entendu, un ou plusieurs substituants R₃, identiques ou différents, peuvent être présents. Dans le cas des hétérocycles, les substituants peuvent être au niveau de l'atome de carbone ou de l'atome d'azote.

L'invention s'étend naturellement aux sels des composés de formules (I), comme par exemple les sels formés, lorsque les composés de formules (I) comportent une fonction amino ou amino guanidine, avec les acides chlorydrique, bromhydrique, nitrique, sulfurique, phosphorique, acétique, trifluoroacétique, formique, propionique, benzoïque, maléique, succinique, tartrique, citrique, oxalique, glyoxylique, aspartique, alcanesulfoniques, tels que les acides méthane ou éthane-sulfonique, arènesulfoniques, tel que les acides benzène ou paratoluène sulfonique et arylcarboxylique, ou lorsque les composés de formules (I) comportent une fonction acide, avec les sels des métaux alcalins ou alcanino terreux ou d'ammonium éventuellement substitué.

Dans un premier groupe préféré, l'invention a pour objet les composés de formule (I) telle que définie précédemment dans laquelle R₁ représente un hydroxyle et G est un radical NH-C(=NH)-NHRc tel que défini précédemment.

Dans un deuxième groupe préféré, l'invention a pour objet les composés de formules (I) telle que définie précédemment dans laquelle R₁ représente un hydroxyle et G est choisi parmi les hétérocycles suivants :

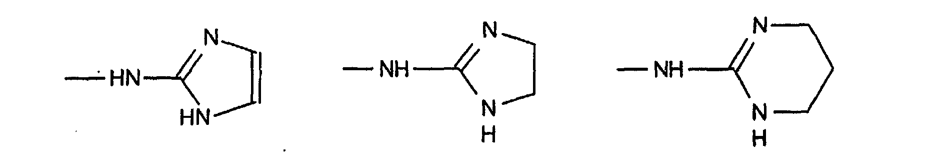

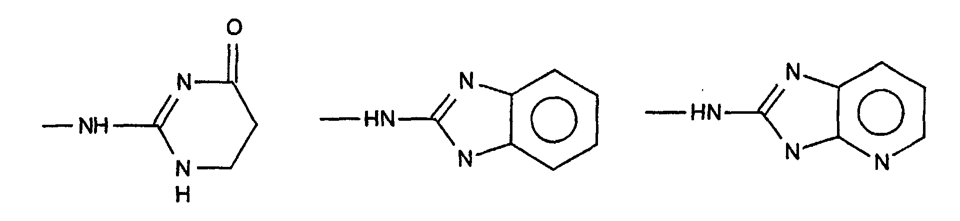

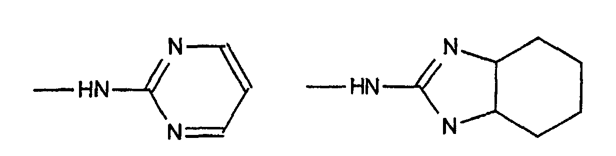

L'invention comprend également toutes les formes tautomères des composés de formule (I) telle que définie plus haut, par exemple en ce qui concerne la forme représentée par la formule (I) avec G représentant 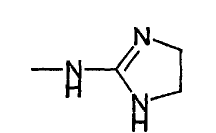 on considère la forme tautomère suivante : 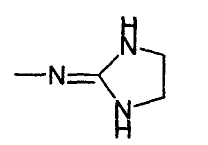 et toutes les autres formes qui diffèrent par la position différente de l'atome d'hydrogène.

Dans un troisième groupe préféré l'invention a pour objet les composés de formule (I) telle que définie précédemment dans laquelle Z est un atome d'hydrogène ou un groupement NHCO₂CH₂Ph, NHCOCH₃, NHCO₂CH₂-adamantyle et G est un groupement :

-NH-C(=NH)-NH₂,

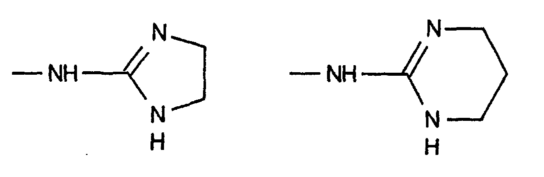

Dans un quatrième groupe préféré l'invention a pour objet les composés suivants :
- 3-[(4,5-dihydro-1H-imidazol-2-yl)hydrazono]-17-oxo-.alpha.-[[(phénylméthoxy)carbonyl]amino]-estra-4,9-diène-11bêta-hexanoate d'éthyle,
- Acide 3-[(4,5-dihydro-1H-imidazol-2-yl)hydrazono]-17-oxo-.alpha.-[[(phénylméthoxy)carbonyl]amino]-estra-4,9-diène-11 bêta-hexanoïque,
- Acide 3-[(4,5-dihydro-1H-imidazol-2-yl)hydrazono]-17-hydroxy-.alpha.-[[(phénylméthoxy)carbonyl]amino]-estra-4,9-diène-11 bêta-hexanoïque,
- Acide 3-[(aminoiminométhyl)hydrazono]-17-oxoestra-4,9-dièn-11 bêta-pentanoique,
- Acide 3-[(1,4,5,6-tetrahydro-2-pyrimidinyl)hydrazono]-17-oxoestra-4,9-diène-11-bêta-hexanoique,
- Acide 3-[(4,5-dihydro-1H-imidazol-2-yl)hydrazono]-17-oxoestra-4,9-diène-11 bêta-hexanoïque,
- Acide 4-chloro-3-[(4,5-dihydro-1H-imidazol-2-yl)hydrazono]-17-oxoestra-4,9-diène-11 bêta-hexanoïque,
- Acide 3-[(aminoiminométhyl)hydrazono]-4-chloro-17-oxoestra-4,9-diène-11 bêta-hexanoïque.
- Acide 3-[(aminoiminométhyl)hydrazono]-17-oxoestra-4,9-dièn-11-bêta-hexanoïque,
- Acide 6-[3-[(4,5-dihydro-1H-imidazol-2-yl)hydrazono]-17-méthylène-estra-4,9-diène-11 bêta-yl] -2-[[(phénylméthoxy)carbonyl]amino]-hexanoïque.

L'os est constamment soumis à un processus dynamique qui inclut la résorption osseuse et la formation osseuse. Ces processus sont médiés via des cellules spécialisées. La formation osseuse est le résultat du dépôt d'une matrice minérale par les ostéoblastes et la résorption osseuse est le résultat de la dissolution de cette matrice osseuse par les ostéoclastes. L'ostéoporose est caractérisée par une perte sèche de cette matrice osseuse. Un ostéoclaste mature activé résorbe l'os après adhésion à la matrice osseuse via la sécrétion d'enzymes protéolyptiques, et de protons à l'intérieur de la zone d'adhésion, aboutissant à des dépressions ou des creusements de la surface de l'os qui apparaissent au moment où l'ostéoclaste se détache de l'os.

Les composés de formule (I) ainsi que leurs sels d'addition pharmaceutiquement acceptables présentent d'intéressantes propriétés pharmacologiques. Ces composés inhibent la résorption osseuse qui est médiée via les ostéoclastes.

Les composés de l'invention sont ainsi utiles dans le traitement de maladies provoquées par la perte de la matrice osseuse, notamment, l'ostéoporose, l'hypercalcemie de malignité, l'ostéopénie due aux métastases osseuses, les parodontites, l'hyperparathyroidisme, les érosions periarticulaires dans l'arthrite rhumatoïde, la maladie de Paget, l'ostéopénie induite par l'immobilisation, les traitements par les glucocorticoïdes ou les déficiences d'hormones sexuelles mâles ou femelles.

Ils peuvent également être utilisés pour le traitement des désordres inflammatoires, cancéreux et cardiovasculaires incluant l'athérosclérose, la resténose.
Ils peuvent enfin être utilisés comme inhibiteurs de l'angiogenèse et donc dans le traitement des tumeurs, par inhibition de leur néovascularisation, des rétinopathies diabétiques et des néphropathies.

Des études récentes ont montré que la fixation de l'ostéoclaste à l'os est médiée par des récepteurs : les intégrines.

Les intégrines sont une superfamille de récepteurs médiant les processus d'adhésion cellule/cellule et plus particulièrement cellule/matrice, incluant notamment α2bβ3 comme récepteur des plaquettes sanguines (fibrinogène) et αvβ3 comme récepteur de la vitronectine, des sialoprotéines osseuses comme l'ostéopontine et la thrombospondine.

Ces récepteurs qui sont des hétérodimères protéiques composés de deux sous unités α et β, possèdent des sites de fixation d'ions divalents comme le Ca²⁺ notamment et un site de reconnaissance de leur ligand prédéfini par la qualité de leurs sous unités.

Le récepteur αvβ3 est une glycoprotéine transmembranaire qui est exprimée dans un grand nombre de cellules incluant les cellules endothéliales, les cellules du muscle lisse, l'ostéoclaste et des cellules cancéreuses ce qui entraîne ainsi une pluripotentialité des composés selon l'invention.

Les récepteurs αvβ3 exprimés au niveau de la membrane des ostéoclastes sont à la base du processus d'adhésion/résorption, contribuent à l'organisation du cytosquelette cellulaire, et sont impliqués dans l'ostéoporose (Ross et al., J. Biol. Chem., 1987, 262, 7703).

Les récepteurs αvβ3 exprimés au niveau des cellules du muscle lisse de l'aorte, stimulent leur migration vers la neointima, ce qui entraîne la formation de l'athérosclérose et la survenue de resténose post-angioplastique (Brown et al, Cardiovascular Res.(1994), 28, 1815).

Les cellules endothéliales secrètent des facteurs de croissance qui sont mitogènes pour l'endothélium et peuvent contribuer à la formation de nouveaux vaisseaux sanguins (angiogenèse). La stimulation angiogénique provoque la formation de nouveaux vaisseaux sanguins.

Les antagonistes de l'intégrine αvβ3 peuvent ainsi entraîner une régression des tumeurs cancéreuses en induisant l'apoptose des vaisseaux sanguins angiogéniques. (Brook et al. Cell (1994) 79, 1157).

Les ligands naturels de l'intégrine αvβ3 contiennent tous le motif RGD (Arg-Gly-Asp). Les peptides contenant ce motif RGD ainsi que des anticorps anti αvβ3 sont connus pour leur capacité d'inhibition de la résorption de la dentine, d'empêchement de l'adhésion des ostéoclastes sur les matrices minéralisées (Horton et al. Exp. Cell. Res. (1991), 195, 368).

Le peptide Echistatine isolé du venin de serpent contenant également un motif RGD est décrit comme inhibiteur de l'adhésion des ostéoclastes à l'os, et est donc un puissant inhibiteur de la résorption osseuse dans les tissus en culture in vitro (Sato et al. J. Cell. Biol. (1990), 111, 1713) et in vivo chez le rat (Fisher et al. Endocrinology (1993), 132, 1441).

Les composés de formule (I) ainsi que leurs sels d'addition et leurs esters pharmaceutiquement acceptables peuvent posséder notamment une affinité vis-à-vis d'autres intégrines ayant pour ligand la vitronectine (αvβ1, αvβ5, α2bβ3) en inhibant la liaison à leur ligand naturel.

Cette propriété rend ainsi les composés de l'invention utilisables pour la prévention ou le traitement de maladies dont la pathologie sous-jacente est provoquée par les ligands ou les cellules qui interagissent avec le récepteur de la vitronectine.

Ces composés peuvent posséder également une activité vis-à-vis d'autres intégrines qui intéragissent avec leur ligand via la séquence tripeptidique RGD, leur conférant des propriétés pharmacologiques utilisables pour traiter les pathologies associées à ces récepteurs.

Cette activité vis-à-vis des intégrines rend ainsi les composés de l'invention utilisable dans le traitement de nombreuses maladies telles que celles mentionnées plus haut ou dans la revue de Dermot Cox DN & P 8(4) Mai 1995, 197-205 dont le contenu est intégré dans la présente demande.

L'invention a donc pour objet les composés de formule (I) à titre de médicaments, ainsi que leurs sels d'addition ou leurs esters pharmaceutiquement acceptables.

L'invention a plus particulièrement pour objet les composés de formule (I), ainsi que leurs sels d'addition pharmaceutiquement acceptables tels que définis précédemment, à titre de médicament ayant une activité antagoniste du récepteur de la vitronectine,.

L'invention à tout particulièrement pour objet les composés de formule (I), ainsi que leurs sels d'addition pharmaceutiquement acceptables tels que définis précédemment, à titre de médicament ayant une activité inhibitrice de la résorption osseuse ou pour le traitement ou la prévention de l'ostéoporose.

L'invention à tout particulièrement pour objet les composés de formule (I), ainsi que leurs sels d'addition pharmaceutiquement acceptables tels que définis précédemment, à titre de médicament ayant une activité inhibitrice de la croissance tumorale ou des métastases cancéreuses.

L'invention à tout particulièrement pour objet les composés de formule (I), ainsi que leurs sels d'addition pharmaceutiquement acceptables tels que définis précédemment, à titre de médicament ayant une activité anti-inflammatoire ou pour le traitement ou la prévention des désordres cardiovasculaires, la resténose, l'artériosclérose, les néphropathies ou rétinopathies.

Parmi les médicaments de l'invention, on peut citer particulièrement les composés décrits dans la partie expérimentale.

La posologie varie en fonction de l'affection à traiter et de la voie d'administration : elle peut varier par exemple de 1 mg à 1000 mg par jour chez l'adulte par voie orale.

L'invention s'étend aux compositions pharmaceutiques renfermant comme principe actif au moins un médicament tel que défini ci-dessus et un ou plusieurs supports, véhicules, diluants ou adjuvants.

Les composés de formule (I) sont utilisés par voie digestive, parentérale ou locale, par exemple par voie percutanée. Ils peuvent être prescrits sous forme de comprimés simples ou dragéifiés, de gélules, de granulés, de suppositoires, d'ovules, de préparations injectables, de pommades, de crèmes, de gels, de microsphères, de nanosphères, d'implants, de patchs, lesquels sont préparés selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I) comprenant les étapes suivantes :
a) On soumet un composé de formule (IIa) : 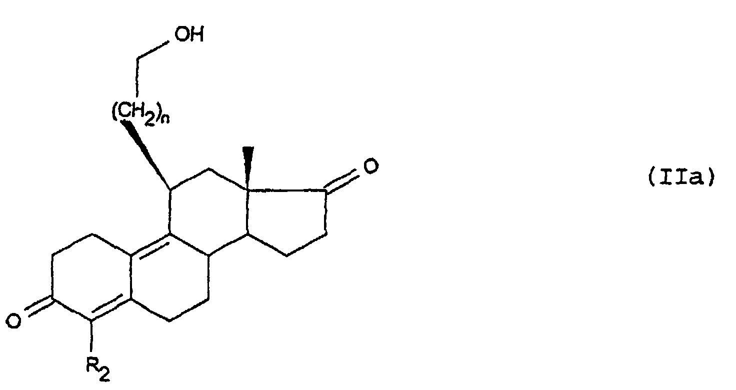 n étant un entier variant de 1 à 6, R₂ étant tel que défini précédemment,
   - d'abord à l'action d'un réactif d'halogénation ou d'un réactif d'activation de l'alcool,
   - puis à l'action en présence de sodium, d'un composé de formule(F2) : 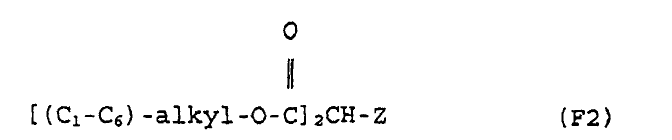
   dans laquelle Z est tel que défini précédemment afin d'obtenir le composé de formule (III) : 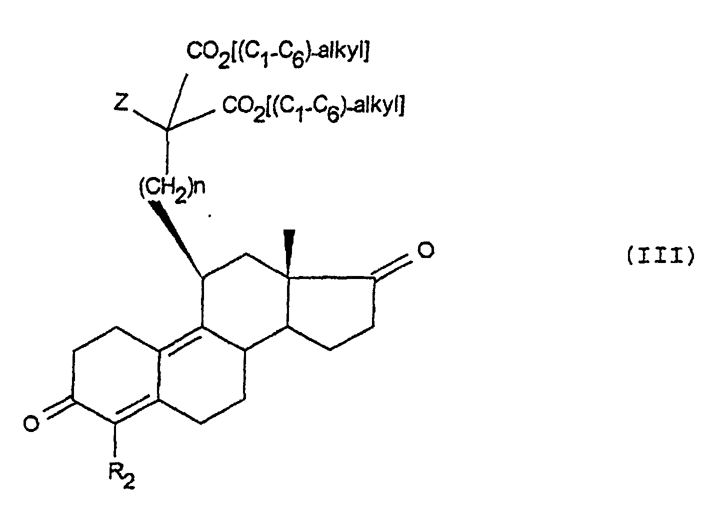
b) on soumet le composé de formule (III), à l'action d'un réactif de saponification puis de décarboxylation afin d'obtenir le composé de formule (IV) : 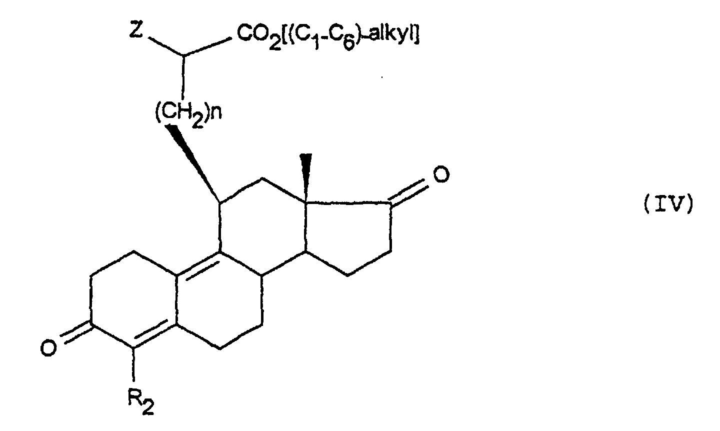
c) On soumet le composé de formule (IV) à l'action d'un composé de formule (F1) :

   G-NH₂ (F1)

   dans laquelle G est tel que défini précédemment afin d'obtenir un composé de formule (I) qui si désiré ou si nécessaire, dans un ordre approprié subit l'une ou plusieurs des réactions suivantes :
   - action d'un réactif d'halogénation en position 4, lorsque R₂ est un atome d'hydrogène,
   - réduction en position 17 puis le cas échéant alkylation ou acylation,
   - introduction du groupement méthylène en position 17,
   - saponification,
   - estérification ou amidification de la fonction acide,
   - salification par un acide ou par une base.

L'invention a notamment pour objet un procédé tel que défini précédemment dans lequel les réactions d'halogénation en position 4, de réduction en position 17 suivie le cas échéant dune alkylation ou d'une acylation ainsi que de l'introduction du groupement méthylène peuvent s'effectuer au niveau des étapes a ou b, c'est-à-dire sur les composés de formules (IIa) ou sur les composés intermédiaires de formule (III) ou (IV).

L'invention a également pour objet un procédé de préparation des composés de formule (I) avec Z représentant un atome d'hydrogène comprenant les étapes suivantes :
a) on soumet un composé de formule (IIb) : 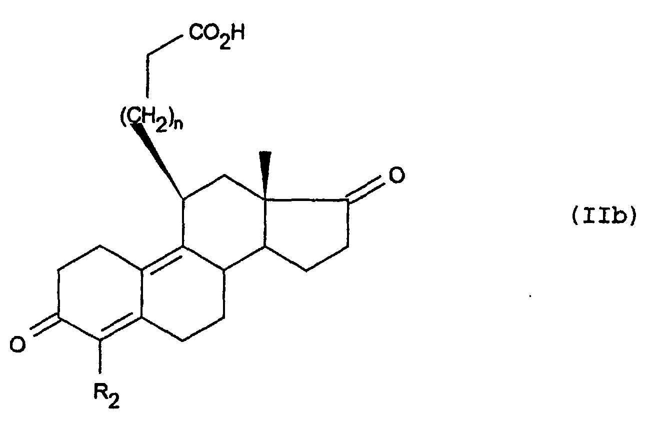 dans laquelle n est un entier variant de 1 à 6 et R₂ est un atome d'hydrogène,
   - le cas échéant à l'action d'un réactif d'halogénation en position 4 afin d'obtenir le composé de formule (IIb) avec R₂ représentant un halogène,
   - puis à l'action d'un composé de formule (F1) :

      G-NH₂ (F1)
   dans laquelle G est tel que défini précédemment afin d'obtenir un composé de formule (I) qui si désiré ou si nécessaire dans un ordre approprié subit l'une ou plusieurs des réactions suivantes :
   - réduction en position 17 puis le cas échéant alkylation ou acylation,
   - introduction du groupement méthylène en position 17,
   - estéritication ou amidification de la fonction acide,
   - salification par un acide ou une base.

A titre de variante, les réactions de réduction en position 17, suivi le cas échéant d'une alkylation ou d'une acylation d'introduction du groupement méthylène en position 17, ainsi que les réactions d'estérification, d'amification ou de salification de la fonction acide peuvent s'effectuer préalablement sur le composé de formule (IIb).

L'action d'un réactif d'halogénation sur l'alcool de formule (IIa) s'effectue de préférence par action de tétrabromure de carbone en présence de triphénylphosphine dans le dichlorométhane. Par action d'un agent activant de l'alcool, on entend de préférence la préparation d'un mésylate, tosylate en triflate selon les méthodes connues de l'homme du métier. L'action du composé de formule (F2) s'effectue notamment en présence de sodium dans l'éthanol. La formation d'un dérivé 4-halogéné des composés de formules (IIa) ou (IIb) s'effectue en particulier par action du N-bromosuccinimide (R₂=H → R₂=Br) ou par action de N-chlorosuccinimide (R₂=H → R₂=Cl) en présence d'un solvant aprotique dipolaire tel que le diméthylformamide. La réduction du 17 céto en alcool correspondant (X=OH et Y = H) s'effectue notamment par action d'un borohydrure alcalin tel que le borohydrure de sodium dans le méthanol ou l'éthanol ou par action de l'hydrure d'aluminium et de lithium.

L'introduction du groupement méthylène en position 17 s'effectue par exemple par action d'un réactif de wittig (Ph₃P=CH₂) sur le composé 17 céto correspondant selon les méthodes usuelles, après avoir protégé la fonction 3-céto. l'action de G-NH₂ (F1) s'effectue soit sans solvant, soit dans un alcool tel que l'éthanol ou le butanol. L'amine G-NH₂ est éventuellement utilisée sous la forme d'un sel tel que le chlorhydrate ou le bromohydrate. Les réactions de décarboxylation, saponification, alkylation, acylation, estérification ou acylation s'effectuent selon les méthodes usuelles connues de l'homme du métier. Les réactions de salification peuvent être effectuées dans des conditions usuelles. On opère par exemple, pour salifier le groupement terminal CO₂H, en présence d'un sel de sodium tel que le carbonate de sodium ou le carbonate acide de sodium ou de potassium. De même, la salification de l'amine ou de l'aminoguanidine que peut représenter G, par un acide, est réalisée dans les conditions usuelles. On opère par exemple avec l'acide chlorhydrique, par exemple en solution éthérée.

Les réactions de protection et de déprotection éventuellement nécessaires au cours des différentes étapes de synthèse sont les méthodes classiques connues de l'homme du métier. Une revue assez complète se trouve dans l'ouvrage suivant : Protective groups in organic synthesis T.W greens, John Wiley & sons (1981).

A titre d'exemple, les réactions de déprotection des composés selon l'invention renfermant le groupement -(CH₂)ₙ-CH₂OP en position 11, lorsque P est un radical méthyle, peuvent s'effectuer par action de tribromoborane dans le dichlorométhane ou d'acide chlorhydrique dans la pyridine ; les réactions de déprotection lorsque P est un groupement benzyle peuvent s'effectuer par action d'hydrogène en présence de palladium sur charbon dans l'acétate d'éthyle ou par action d'acide trifluoroacétique, les réactions de déprotection lorsque P est un groupement tertbutyldiphénylsilyle peuvent s'effectuer par action de fluorure de tétrabutyl ammonium en solution dans le tétrahydrofuranne. Lorsque P est un groupement tétrahydropyrannyle, la déprotection s'effectue en présence d'un acide aqueux dans un solvant alcoolique et de préférence par action de l'acide chlorhydrique dans le méthanol.

Les composés de formule (IIa) et (IIb) avec R₂ représentant un atome d'hydrogène sont connus et décrits dans la demande de brevet Européen EP-A-0384842.

Les composés de formules (F1) et (F2) sont commerciaux, connus ou accessibles à l'homme du métier.

L'invention a également pour objet les composés de formules (III) et (IV) ainsi que les composés de formules (IIa) et (IIb) dans lesquelles R₂ est un atome d'halogène tels que définis plus haut.

Les exemples suivants illustrent l'invention sans toutefois la limiter.

### EXEMPLE 1: 3-[(4,5-dihydro-1H-imidazol-2-yl)hydrazono]-17-oxo-.alpha.-[[(phénylméthoxy)carbonyl]amino]-estra-4,9-diène-11 bêta-hexanoate d'éthyle.

### Stade A : Bromation 11 bêta-(4-bromobutyl)-estra-4,9-diène-3,17-dione

A une solution de 1,027 g de 11 bêta-(4-hydroxybutyl)-estra-4,9-diène-3,17-dione dans 10 ml de dichlorométhane, on ajoute 1,094 g de tétrabromure de carbone et 0,865 g de triphénylphosphine en plusieurs fois et agite une heure à environ 26°C. Après purification par chromatographie on obtient 0,816 g de produit attendu.
IR (CHCl₃)
C=O 1736 cm⁻¹, diénone 1657 et 1604 cm⁻¹,

### Stade B : Introduction de F2 = (CO₂Et)₂CH-NHCO₂CH2Ph [4-[3,17-dioxo-estra-4, 9-diène-11 bêta-yl]butyl]-[[(phénylméthoxy)carbonyl]amino]-propanedioate de diéthyle.

A une solution renfermant 77 mg d'hydrure de sodium à 50% et 0,5 g de [[(phénylméthoxy)carbonyl]amino]-propanedioate de diéthyle dans 4 ml de THF et 0,5 ml de DMF on ajoute 439 mg du produit obtenu au stade précédent dans 1 ml d'acétonitrile et 1,5 équivalent de iodure de sodium, puis porte au reflux pendant 2 heures. Après purification par chromatographie, on obtient 0,302 g de produit attendu.
IR (CHCl₃)
=C-NH 3417 cm⁻¹ ; C=O 1756, 1736, 1721, 1656 cm⁻¹ ;
C=C + aromatique + amide II 1604, 1497 cm⁻¹

### Stade C : Saponification/Decarboxylation 3,17-dioxo-.alpba.-[[(phénylméthoxy)carbonyl]amino]-estra-4,9-diène-11 bêta-hexanoate d'éthyle.

A une solution de 0,29 g de produit obtenu au stade précédent dans 5 ml d'éthanol on ajoute 0,5 ml de soude 2N, agite pendant 3 heures puis acidifie par 0,5 ml d'acide chlorydrique 2N. On ajoute 10 ml de dioxane, porte 2 heures à 100°C et purifie par chromatographie en éluant avec le mélange cyclohexane/acétate d'éthyle 6/4. On obtient 155 mg de produit pur attendu.
IR (CHCl₃)
-NH 3434 cm⁻¹ ; C=O 1735,1722 cm⁻¹ ; diénone C=O 1657 cm⁻¹
C=C 1603 cm⁻¹, amide II +aromatique 1509 cm⁻¹

### Stade D : Introduction de G-NH₂ 3-[(4,5-dihydro-1H-imidazol-2-yl)hydrazono]-17-oxo-.alpha.-[[(phénylméthoxy)carbonyl]amino]-estra-4,9-diène-11 bêta-hexanoate d'éthyle

A une solution de 0,956 g de produit obtenu au stade précédent dans 10 ml d'éthanol, on ajoute 0,386 g de bromohydrate de 2-hydrazino-2-imidazoline, porte au reflux pendant 3 heures et purifie par chromatographie en éluant avec le mélange CH₂Cl₂/MeOH 90/10. On obtient 1,06 g de produit brut attendu.
IR (CHCl₃)
NH 3446 cm⁻¹ ; C=O 1734 cm⁻¹ ; C=N, C=C, amide II 1674, 1629, 1565, 1509, 1488 cm⁻¹

### EXEMPLE 2 : Acide 3-[(4,5-dihydro-1H-imidazol-2-yl) hydrazono]-17-oxo-.alpha.-[[(phénylméthoxy)carbonyl]amino]-estra-4,9-diène-11 bêta-hexanoïque

### Méthode a)

A une solution de 0,904 g du produit obtenu au stade D de l'exemple 1 dans 5 ml d'éthanol on ajoute 1,4 ml de soude 2N et agite à température ambiante pendant 1 heure. On ajoute 1,4 ml d'acide chlorydrique 2N, purifie par chromatographie en éluant avec le mélange CHCl₃/MeOH/NH₄OH 80/20/2 et obtient 0,520 g de produit attendu sous la forme d'un mélange ΔE/ΔZ 70/30.

### Méthode b)

### Stade A : Saponification/décarboxylation

A une solution de 3 g de l'ester obtenu au stade C de l'exemple 1 dans 60 ml d'éthanol, on ajoute 5 ml de soude 2N et agite 1 heure à 20°C puis acidifie par 5 ml d'acide chlorhydrique 2N ; On ajoute 20 ml de dioxane, porte 3 heures à 120°C, évapore les solvants, reprend dans 60 ml d'éthanol, ajoute de nouveau 5 ml de soude 2N. Après évaporation des solvants, le produit brut est purifié par chromatographie en éluant avec le mélange CHCl₃/MeOH NH₄OH 8/2/0,4. On obtient 1,06 g de produit attendu.

### Stade B : Introduction de G-NH₂

On opère comme à l'exemple 1 stade D à partir de 266 mg du produit obtenu au stade précédent et 135 mg de bromohydrate de 2-hydrazino-2-imidazoline. On obtient 109 mg de produit attendu.
RMN (CDCl₃)
0,91 et 1,04 18-Me ; 3,77 CH₂-N ; 4,19 CO-CH-NH ; 5,09 OCH₂Ph ; 5,75 et 6,54 H4 dédoublement 35%-65% ( ΔE majoritaire) ; 7,34 phényle ; 5,78, 6,07-6,54 ; 11,65-11,98 H mobile.

### EXEMPLE 3 - Réduction de la fonction 17 céto Acide 3-[(4,5-dihydro-1H-imidazol-2-yl)hydrazono]-17-hydroxy alpha.-[[(phénylméthoxy)carbonyl]amino]-estra-4,9-diène-11 bêta-hexanoïque

On agite pendant 1 heure à température ambiante le mélange constitué de 0,208 g du produit de l'exemple 2, 14 mg de borohydrure de sodium et 3,5 ml d'éthanol, ajoute 3,5 ml d'acide chlorhydrique 1N, agite 10 minutes, évapore le solvant, purifie par chromatographie le produit obtenu en éluant avec le mélange CHCl₃/MeOH/NH₄OH 80/20/2.
On obtient 0,145 g de produit attendu.
SM : 616⁻= [M-H]⁻ ; 508⁻ = [M-OCH₂Ph] ; 618⁺ = MH⁺ ; 640⁺ = MNa⁺

### EXEMPLE 4 : Acide 3-[(amino iminométhyl)hydrazono]-17-oxoestra-4,9-dièn-11 bêta-pentanoïque

### Stade A : Oxydation Acide 3,17-dioxo-estra-4,9-diène-11 bêta-pentanoique

On ajoute en 22 minutes à une température comprise entre 0° et -4°C, 7 ml du réactif de Heilbron-Jones (1,89 g de CrO₃) à 2,9 g de 11 bêta-5-(hydroxypentyl)-estra-4,9-diène-3,17-dione dans 140 ml d'acétone, agite 5 mn à 0°C puis ajoute 2,5 ml de méthanol, 22 g de carbonate de baryum et 220 ml d'eau puis agite vigoureusement 1 heure à température ambiante. Après essorage, extraction de la phase aqueuse avec du dichlorométhane, lavage et séchage, on évapore sous pression réduite jusqu'à obtention de 3,4 g de produit attendu.
Rf : (dichlorométhane/acétone 80/20) = 0,15
IR (CHCl₃)
C=O 1736, 1709, 1657 ; C=C 1602 cm⁻¹

### Stade B - Introduction du G-NH₂ Acide 3-[(amino iminométhyl)hydrazono]-17-oxoestra-4,9-dièn-11 bêta-pentanoïque

On porte au reflux pendant 2 heures le mélange constitué de 480 mg de l'acide obtenu au stade précédent, 285 mg de chlorhydrate d'aminoguanidine et 10 ml d'éthanol, évapore sous pression réduite jusqu'à obtention du produit brut que l'on purifie par chromatographie en éluant avec le mélange CH₂Cl₂/MeOH /NH₄OH 40/10/2 et qui est ensuite lyophilisé. On obtient 170 mg de produit attendu sous forme d'un mélange ΔZ/ΔE 40/60
RMN (DMSO 300MHz)
0,96 18Me ; 5,82(s) H4 ΔE ; 6,65(s) H4 ΔZ ; 6,8 7,5 absorptions larges et mobiles 3H

### Example 5 : Acide 3-[(amino-iminométhyl)hydrazono]-17-oxoestra-4,9-diène-11 bêta-hexanoïque

On opère comme à l'exemple 4 stade B mais à partir de 380 mg d'acide 3,17-dioxo-estra-4,9-diène-11beta-hexanoïque et 220 mg de chlorhydrate d'amino guanidine dans 10 ml d'éthanol. On obtient 50 mg de produit insoluble blanc correspondant à l'isomère ΔE et 130 mg de lyophilisat beige correspondant à l'isomère ΔZ
RMN (isomère ΔZ) (DMSO, 300MHz)
18 Me 1,00 (s) ; H4 6,73(s), H mobiles 6,89, 8,17
RMN (isomère ΔE) (DMSO, 300MHz)
18-Me 0,97 (s) ; H4 5,82(s), H mobiles 6,5 à 7,6

### EXEMPLE 6 : Acide 3-[(1,4,5,6-tetrahydro-2-pyrimidinyl)-hydrazono]-17-oxoestra-4,9-diène-11-bêta-hexanoïque

On opère comma à l'exemple 4 stade B mais à partir de 0,597 g d'acide 3,17-dioxo-estra-4,9-diène-11 bêta-hexanoïque et 0,453 mg de bromhydrate de 2-hydrazono-1,4,5,6-tétrahydropyrimidine dans 12,5 d'éthanol. On isole 3 fractions correspondant à différentes proportions de ΔE/ΔZ
a) 65 mg b) 99 mg et c) 65 mg
RMN (CDCl₃, 300MHz)
Fraction b : Mélange ΔE/ΔZ 90/10
1,04(s), 1,06(s) 18-Me ; 3,42(m) CH₂-N ; 5,76(s) H4 ΔE 6,67(s)H4 ΔZ ; 6,64(s) 12,40(m) H mobiles ; 1,20 à 3,20(m) squelette stéroïde
Fraction c : Mélange ΔE/ΔZ 60/40
1,04(s) 1,06(s) 18-Me ; 3,42(m) CH₂N ; 5,77(s) 6,66 (manqué) H4 6,66(s) H mobile ; 1,24 à 3,05(m) squelette stéroïde ; 11,8(m) H mobile

### EXEMPLE 7 : Acide 3-[(4,5-dihydro-1H-imidazol-2-yl)-hydrazono]-17-oxo-estra-4,9-diène-11 bêta-hexanoïque

On opère comme à l'exemple 4 stade B mais à partir de 0,5 g d'acide 3,17-dioxo-estra-4,9-diène-11 bêta-hexanoïque, 0,353 g de bromohydrate de 2-hydrazino-2-imidazoline dans 12,5 ml d'éthanol. On obtient 220 mg de produit pur attendu (mélange ΔE/ΔZ 85/15).
IR (CHCl₃)
C=O 1733 cm⁻¹ ; C=O et/ou C=N 1661 cm⁻¹ ; C=N, C=C 1617, 1597, 1546 cm⁻¹
RMN (DMSO, 300 MHz)
0,97(s) 18-Me ; 3,40(sl) CH₂-N ; 5,80(s)H4 ΔE ; 6,60(s) H4 ΔZ ; 6,53(m) 6,86(m) H mobile ; 1,16 à 3,00 squelette stéroïde.

### EXEMPLE 8 : Acide 4-chloro-3-[(4,5-dihydro-1H-imidazol-2-yl)hydrazono]-17-oxo-estra-4,9-diène-11 bêta-hexanoïque.

### Stade A : Acide 4-chloro-3,17-dioxo-estra-4,9-diène-11 bêta-hexanoïque

On ajoute sous atmosphère d'azote, à environ 54°C, 382 mg de N-chlorosuccinimide à 829 mg d'acide 3,17-dioxoestra-4,9-diène-11beta-hexanoïque dans 10 ml de diméthylformamide, agite 10 minutes à environ 61°C, puis, après refroidissement, verse une solution aqueuse de chlorure de sodium, extrait, lave et évapore sous pression réduite jusqu'à obtention du produit brut que l'on purifie par chromatographie en éluant avec le mélange dichlorométhane/acétone 8/2 pour obtenir 387 mg de produit attendu.
IR (CHCl₃)
C=O 1736, 1712, 1673 cm⁻¹ ; C=C 1587, 1551 cm⁻¹

### Stade B : Condensation Acide 4-chloro-3-[(4,5-dihydro-1H-imidazol-2-yl)hydrazono]-17-oxo-estra-4,9-diène-11 bêta-hexanoïque.

On mélange 159 mg de produit chloré préparé au stade précédent et 137 mg de bromohydrate de 2-hydrazino-2-imidazoline dans 4 ml d'éthanol. Après traitement et purification, on obtient 187 mg de produit attendu.
IR (CHCl₃)
=C-NH 3449 cm⁻¹ + absorption générale ; C=O 1735 cm⁻¹ ;
C=N + C=C + CO₂⁻, : 1672, 1620, 1604, 1546, 1513 cm⁻¹.

### EXEMPLE 9 : Acide 3-[(aminoiminométhyl)hydrazono]-4-chloro-17-oxo-estra-4,9-diène-11 bêta-hexanoïque.

On opère comme au stade B de l'exemple 8, à partir de 380 mg du produit chloré préparé à l'exemple 8 stade A et 201 mg de chlorhydrate d'aminoguanidine. On obtient 77 mg de produit attendu.
IR (Nujol)
Absorption OH/NH ; C=O 1731 cm-1 ; Système conjugué + NH2 1676, 1604, 1532 cm⁻¹

### Exemple 10 : Acide 6-[3-[(4,5-dihydro-1H-imidazol-2-yl)-hydrazono]-17-méthylène-estra-9,9-diène-11 bêta-yl]-2-[[(phénylméthoxy)carbonyl]amino]-hexanoïque

### Stade A : Blocage de l'alcool avec le dihydropyrane 11beta-[4-[(tetrahydro-2H-pyran-2-yl)oxy]butyl-estra-4,9-diène-3,17-dione

On agite pendant 3 heures une solution contenant 3,42 g de 11 bêta-(4-hydroxybutyl)-estra-4,9-diène-3,17-one, 20 ml d'éther, 9 ml de dihydropyrane et de l'acide paratoluène sulfonique en quantité catalytique puis ajoute 10 cm³ de triéthylamine, évapore sous pression réduite les solvants et purifie par chromatographie en éluant avec le mélange dichlorométhane/acétone 90/10. On obtient 3,70 g de produit pur attendu (Rf=0,42).
IR (CHCl₃) : C=O 1736, C=C 1657 cm⁻¹ ; 1603 cm⁻¹
RMN (CDCl₃)
1,08(s) 18Me ; 3,08(pp) H11 ; 3,3 (dt) 3,73(m) CH₂O ;
3,51(m)-3,84(m) CH₂O du THP ; 4,55 H angulaire du THP ; 5,71(s) H4

### Stade B : Blocage de la cétone en 3 sous forme de méthyloxime et déblocage de l'OH primaire 3-(O-méthyloxime) de 11bêta-(4-hydroxybutyl)-estra-4,9-diène-3,17-one

On agite à température ambiante pendant 18 heures le mélange constitué de 0,98 g de dérivé préparé au stade précédent, 0,215 g de chlorhydrate de méthylhydroxylamine, 10 ml de méthanol, 0,160 g d'acétate de sodium et 2 ml d'eau. Le mélange réactionnel est ensuite dilué par 50 ml d'eau puis extrait au dichlorométhane. On récupère 0,874 g de résine jaune que l'on purifie par chromatographie en éluant avec le mélange cyclohexane/acétate d'éthyle 7/3.
Rf: 0,17 isomère ΔE et 0,12 isomère ΔZ.
On récupère :
a)le produit ayant un Rf de 0,17 m=0,382 g
   IR (CHCl₃)
   OH 3624 cm⁻¹
   C=O 1734 cm⁻¹ 17 céto
   C=N, C=C 1605 cm⁻¹
   RMN isomère ΔE (CDCl₃)
   1,06(s) 18Me ; 3,64(t) CH₂O ; 2,99 H11 ; 3.88 (s) OMe ; 5,80 (s) H4 ΔE
b)le produit ayant un Rf de 0,12 m=0,211 g
   IR ( CHCl₃)
   OH 3624 cm⁻¹
   C=O 1734 cm⁻¹ 17 céto
   C=C, C=N 1603 cm⁻¹
   RMN isomère ΔZ CDCl₃
   1,06(s) 18Me ; 3,01(q1) H11 ; 3,64(t) CH₂O ; 3,87(s) OMe ; 6,36(s) H4 ΔZ

### Stade C : Réaction de Wittig : Introduction du groupement méthylène en 17 3-(O-méthyloxime) de 11beta-(4-hydroxybutyl)-17-méthylèneestra-4,9-diène-3-one

On agite sous pression réduite à 100°C pendant 40 minutes, le mélange constitué de 0,635 g de bromure de triphényl méthyl phosphonium et 0,199 g de ter butylate de potassium. Après avoir refroidi à température ambiante et mis sous azote, on introduit 5 ml de tétrahydrofurane, et 0,162 g du stéroïde préparé au stade précédent (Δ E). La solution est portée à reflux pendant 3 heures puis refroidie, hydrolysée par une solution de chlorure d'ammonium et extraite. On obtient 0,480 g de résine jaune que l'on purifie par chromatographie en éluant avec le mélange cyclohexane/acétate d'éthyle 7/3 (Rf=0,10). On récupère 0,130 g d'huile incolore. IR (CHCl₃)
OH 3624 cm⁻¹
C=C, C=N 1654 cm⁻¹
RMN ( CDCl₃)
0,96(s) 18Me ; 3,65(t) CH₂O ; 3,87(s) OMe ; ∼4,60 CH₂=C ; 5,77(s) H4

### Stade D : Déblocage de la cétone en 3 11bêta-(4-hydroxybutyl)-17-méthylène-estra-4,9-dièn-3-one

On agite à température ambiante pendant 24 heures une solution contenant 0,188 g de produit préparé au stade précédent, 2 ml d'acétone et 0,5 ml d'acide chlorhydrique 6N. On dilue à l'eau puis extrait au dichlorométhane. On obtient 0,174 g de résine que l'on purifie par chromatographie en éluant avec le mélange cyclohexane/acétate d'éthyle 7-3. On récupère une résine jaune pure m= 0,076 g. (Rf = 0,10)
IR ( CHCl₃)
OH 3625 cm⁻¹
C=O 1654 cm⁻¹
C=C 1603 cm⁻¹

### Stade E : Passage au bromé puis condensation de la chaîne [4-(17-méthylène-3-oxo-extra-4,9-dièn-11betayl)butyl][[(phénylméthoxy)carbonyl]amino]propanedioate de diéthyle

A une solution contenant 0,470 g de stéroïde préparé au stade précédent, 5 ml de dichlorométhane et 0,685 g de tétrabromure de carbone, on ajoute par petites fractions 0,543 g de triphénylphosphine, agite la solution pendant 30 minutes, concentre à siccité puis purifie par filtration sur silice. On obtient 0,340 g de résine jaune qui correspond au dérivé bromé.

Dans une suspension d'hydrure de sodium à 50 % dans l'huile (0,1 g) dans 0,5 ml de DMF et 2,5 ml de THF anhydre on introduit lentement une solution de 0,640 g de [[(phénylméthoxy)carbonyl]amino]-propanedioate de diéthyle en solution dans 1 cm³ de THF. Après 30 mn, on introduit le dérivé bromé obtenu plus haut en solution dans 1 ml de THF. On ajoute alors 0,310 g d'iodure de sodium et 1 ml d'acétonitrile, porte à reflux pendant 3 heures. Le mélange réactionnel est hydrolysé par une solution de phosphate monosodique glacée puis extrait au dichlorométhane. On obtient 1,2 g de résine jaune que l'on purifie par chromatographie sur silice en éluant avec le mélange dichlorométhane/acétone 97/3. On récupère 0,164 g d'huile jaune.
IR (CDCl₃)
Peu ou pas d'OH
NH 3418 cm⁻¹
C=O 1756 ép, 1736 max, 1724 ép, 1654 cétone conj
C=C 1603 max
Et 1589 ép
Amide II 1498

### Stade F : Saponification, décarboxylation puis formation de l'imino guanidine 6-[3-[(4,5-dihydro-1H-imidazol-2-yl)hydrazono]-17-méthylèneestra-4,9-dièn-11beta-yl]-2-[[(phenylméthoxy)carbonyl]amino]-hexanoate d'éthyle.

### a) Saponification-décarboxylation

On agite à température ambiante pendant une heure une solution contenant 0,148 g du dérivé préparé au stade précédent, 3 ml d'éthanol et 0,23 ml de soude 2N, puis acidifie par 0,23 ml d'acide chlorhydrique 2N, agite pendant 10 minutes et concentre à siccité.
Rf = 0,52 éluant dichlorométhane/méthanol/ammoniaque 80/20/4 b)Formation de l'imino guanidine : Introduction de G-NH₂

Le produit brut obtenu ci-dessus est repris dans 3 ml d'isopropanol en présence de 0,1 g d'hydrazone de 2-imidazolidinone et d'une quantité catalytique d'acide paratoluène sulfonique. On porte à reflux pendant 1 heure puis concentre à siccité et purifie par chromatographie éluant (dichlorométhane/méthanol/ammoniaque 80-20-4). On récupère 0,089 g de produit attendu.
IR (CHCl₃)
NH 3446 cm⁻¹, absence de diénone

### Stade G : Saponification de l'ester éthylique - Acide 6[3-[(4,5-dihydro-1H-imidazol-2-yl)hydrazono]-17-méthylène-estra-4,9-diène-11 bêta-yl]-2-[[(phénylméthoxy)-carbonyl]amino]-hexanoïque

On agite à température ambiante pendant une heure une solution contenant 86 mg de l'ester éthylique préparé au stade précédent, 2 ml d'éthanol et 0,3 ml de soude 2N. On neutralise par addition de 0,3 ml d'acide chlorhydrique 1 N, agite pendant 20 mn puis concentre à siccité. Le produit brut obtenu est purifié par chromatographie sur silice en éluant avec le mélange dichlorométhane/méthanol/ammoniaque 80/20/2. On obtient 47 mg de produit attendu.
S.M. : Structure de masse moléculaire 613.
614⁺= [M+H]⁺ 612⁻= [M-H]⁻ 636⁺= [M+Na]⁺

### Compositions pharmaceutiques

Comprimés répondant à la formule suivante :
- produit de l'exemple 2 50 mg
- excipient (talc, amidon, stéarate de magnésium) QS pour un comprimé terminé à 120 mg

### Etude pharmacologique des produits de l'invention

### 1- Etude par les produits de l'invention du déplacement de la liaison : vitronectine/récepteur vitronectine (αᵥβ₃) protocole

Des plaques 96 puits MaxiSorp sont coatées une nuit à 4°C, avec 100 µl de Vitronectine humaine (cf Yatohgo et al. Cell., Structure and fraction 13 : 281-292 (1988) à 2µg/ml, (dilution en tampon de coating).
Le lendemain, les puits sont vidés et les ligands (vitronectine) sont ensuite fixés (voir tampon de fixation) pendant 1H à température ambiante sous agitation douce.
Les puits sont lavés six fois (voir tampon de lavage), puis on ajoute par puits et dans cet ordre :
- 40 µl de tampon d'incubation,
- 10 µl de la dilution du produit à tester,
   (les produits sont dilués dans un mélange 50/50 de DMSO-H₂O)
- 20 µl de récepteur αᵥβ₃ humain (cf Pytela et al.Methods Enzymol (1987) 144:475) (dilution en tampon d'incubation, à adapter suivant le lot de récepteur et selon le ligand).
Le ligand, le récepteur αᵥβ₃ humain et les produits à étudier sont incubés pendant 3 heures à température ambiante sous agitation douce.
Les puits sont à nouveau lavés six fois, puis incubés pendant 2 heures à température ambiante sous agitation douce, en présence de 100 µl d'anticorps 4B12-HRP, anti-récepteur couplé à une peroxydase (l'anticorps 4B12-HRP est dilué en tampon d'incubation. La dilution est à adapter suivant le lot de récepteur).
Les puits sont ensuite lavés six fois avant la mesure de liaison ligand-récepteur faite par l'intermédiaire d'un kit révélateur de peroxydase (TMP Microwell Peroxydase Substrate System Kirkegaard : Réf. cat. 50-76-00).
Ce kit contient un flacon A de substrat (3,3',5,5'-tétraméthylbenzidine à 0,4 g/l) et un flacon B (H2O2 à 0,02 % en tampon Citrate/Acide citrique). Extemporanément, un volume de A est mélangé à un volume de B, puis le mélange réactionnel est distribué à raison de 100 µl/puits. La réaction enzymatique se développe en 12' pour Vitronectine/αᵥβ₃, puis son évolution est stoppée par l'addition de 100 µl d'acide phosphorique 1M.
La densité optique est mesurée à 450 nm.
Tampons :
- tampon de coating : Carbonate 0,05 M, NaOH pH 9,6
- tampon de fixation : PBS contenant 0,5 % de BSA (pH 7,4)
- tampon de lavage : PBS contenant 0,05% de Tween 20 (pH 7,4)
- tampon d'incubation :
   50 mM TRIS pH 7,4
   0,5 % BSA
   0,05 % Tween 20
   1 mM MnCl₂
   50 µM CaCl₂
   50 µM MgCl₂
   100 mM NaCl₁

### Expressions des résultats :

On trace la courbe suivante : le pourcentage de liaison de la vitronectine humaine en fonction du logarithme de la concentration de chaque produit testé.
Pour chaque produit on détermine l'IC₅₀ suivant la formule suivante :
IC₅₀ = (BO + Bmin)/2
BO = Maximum de liaison en l'absence de tout produit
Bmin = Minimum de liaison en présence de la concentration la plus élevée de produit.

### RESULTATS :

| **Exemples** | **Test de compétition binding Vn/VnR ((α**_{**V**}**β**_{**3**}**) IC**_{**50**} **en pM** |
|---|---|
| 4 | 0,133 |
| 5a ΔE | 0,054 |
| 5b ΔZ | 0,375 |
| 9 | 1,95 |
| 8 | 0,017 |
| 7 | 0,025 |
| 6b 60/40 | 0,038 |
| 6a 90/10 | 0,020 |
| 2 | 0,030 |
| 3 | 0,070 |

## Revendications

1. Composés de formule générale (I) : 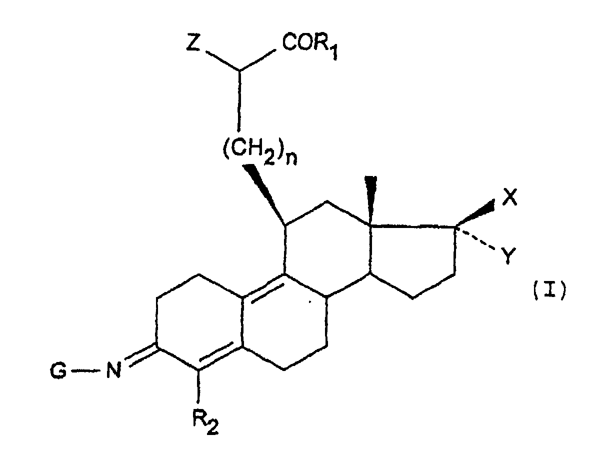 dans laquelle :
- soit X et Y forment ensemble avec le carbone qui les porte un groupement C=O ou C=CH₂,
- soit X représente un radical hydroxy, (C₁-C₆)-alkyloxy ou (C₁-C₆)-alkylcarbonyloxy, le reste alkyle étant non substitué ou substitué par R₃, et Y est un atome d'hydrogène ;
- R₁ représente un radical hydroxy, (C₁-C₆)-alkyloxy, amino, (C₁-C₆)-alkylamino ou (C₂-C₁₂)-dialkylamino, le reste alkyle étant non substitué ou substitué par R₃;
- R₂ représente un atome d'hydrogène ou d'halogène ;
- Z représente un atome d'hydrogène, un groupement NHSO₂Ra, NHCO₂Ra, NHCORa, NHSO₂NHRa ou NHCONHRa ;
- G représente :
- soit un radical 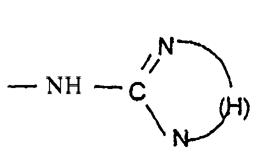 (radical G1) dans lequel (H) forme avec le motif -N=C-N- le reste d'un hétérocycle, choisi parmi 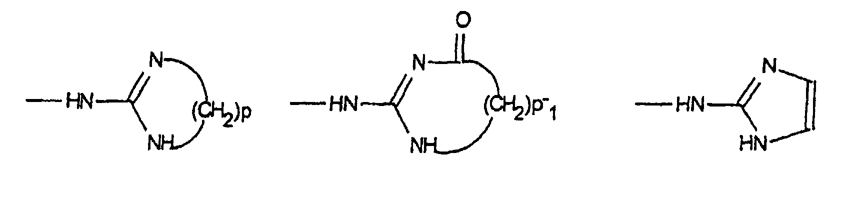 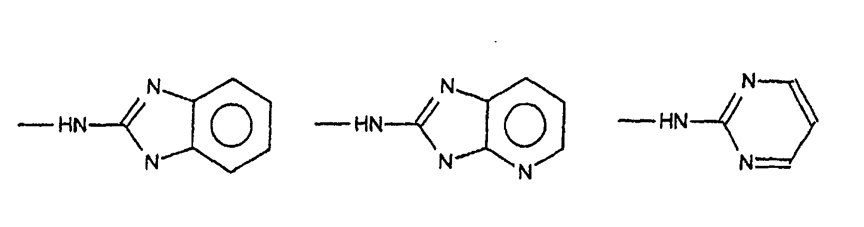 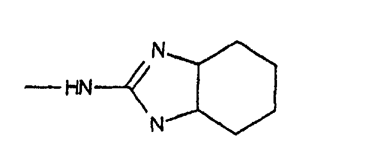
dans lesquels p représente un entier de 1 à 4 et de préférence 2 ou 3.
- soit un radical NRbRc (radical G2),
- soit un hétérocycle (radical G3), aromatique ou non aromatique, saturé ou non saturé comportant de 1 à 9 atomes de carbone et de 1 à 5 hétéroatomes choisis parmi les atomes d'oxygène, d'azote et de soufre.
- soit un radical NRb-C(=A)-NHRc (radical G4) dans lequel A est un atome de soufre, d'oxygène ou NH,
- soit un radical NRb-SO₂Rc (radical G5),
- Ra, Rb et Rc, identiques ou différents, représentent un atome d'hydrogène, un radical -(CH₂)ₘ-Alk ou -(CH₂)ₘ-Ar,
- le terme Alk représentant un radical dérivé d'un hydrocarbure, linéaire, ramifié ou cyclique, saturé ou insaturé, non aromatique renfermant de 1 à 12 atomes de carbone, substitué par R₃ ou non substitué, Ar représentant un phényle substitué par R₃ ou non substitué,
- n est un entier variant de 1 à 6, m représente 0,1,2 ou 3,
- le substituant R₃ représente :
- halogène, oxo, cyano, nitro, formyl, carboxy, (C₁-C₆)-alkyloxycarbonyl, carboxamide,
- un radical alkyle, alkényle ou alkynyle renfermant de 1 à 6 atomes de carbone éventuellement substitués par un ou plusieurs atomes d'halogène,
- un radical cycloalkyle renfermant de 3 à 12 atomes de carbone
- un radical alkoxy ou alkylthio renfermant de 1 à 6 atomes de carbone,
- un radical amino, alkylamino renfermant de 1 à 6 atomes de carbone ou dialkylamino renfermant de 2 à 12 atomes de carbone éventuellement sous forme oxydée,
- un radical aminoalkyle renfermant de 1 à 6 atomes de carbone ou dialkylaminoalkyle renfermant de 3 à 8 atomes de carbone,
- un radical dialkylaminoalkyloxy renfermant de 3 à 18 atomes de carbone,
- un radical hydroxyle éventuellement acylé renfermant de 1 à 12 atomes de carbone,
- un radical acyle renfermant de 1 à 12 atomes de carbone,
- un radical phényle, phénylalkyle ou phényloxy, ces radicaux étant eux-mêmes éventuellement substitués par un ou plusieurs substituants cités plus haut,
lesdits composés de formules (I) étant sous toutes leurs formes stéréoisomères possibles, isolées ou en mélange, ainsi que leurs esters et leurs sels d'addition avec les acides et les bases pharmaceutiquement acceptables.

2. Composés de formule (I) selon la revendication 1 dans laquelle R₁ représente un hydroxyle et G est un radical -NH-C(=NH)-NHRc, ainsi que leurs sels d'addition pharmaceutiquement acceptables.

3. Composés de formule (I) selon la revendication 1 dans laquelle R₁ représente un hydroxyle et G est choisi parmi les hétérocycles suivants : 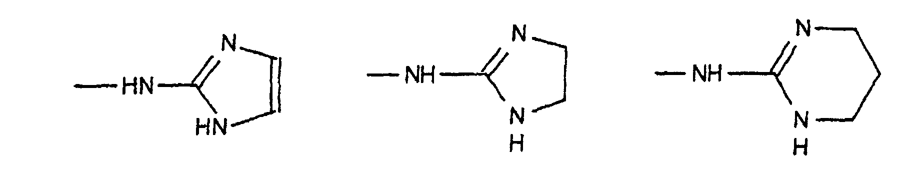 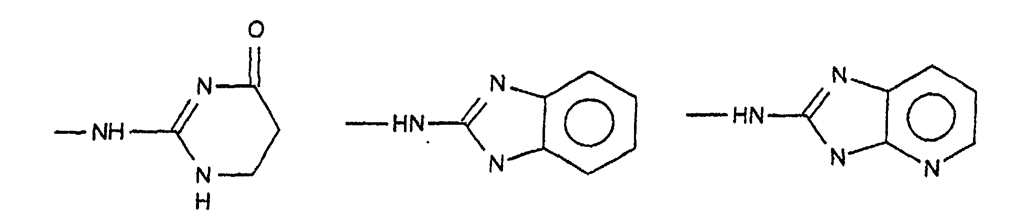 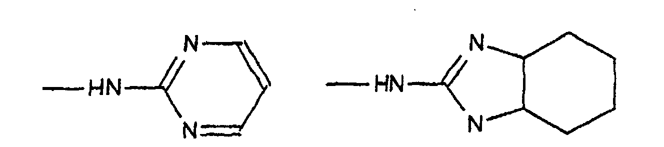 ainsi que leurs sels d'addition pharmaceutiquement acceptables.

4. Composés de formule (I) selon la revendication 1 dans laquelle Z est un atome d'hydrogène ou un groupement -NHCO₂CH₂Ph, -NHAc, -NHCO₂CH₂-adamantyle et G est un groupement
-NH-C(=NH)-NH₂, 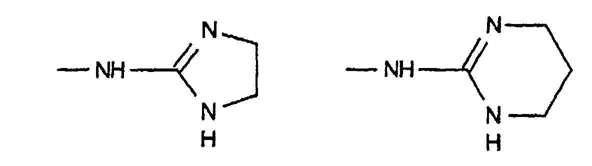
ainsi que leurs sels d'addition pharmaceutiquement acceptables.

5. Composés de formule (I) selon la revendication 1 dont les noms suivent :
- 3-[(4,5-dihydro-1H-imidazol-2-yl)hydrazono]-17-oxo-.alpha.-[[(phénylméthoxy)carbonyl]amino]-estra-4,9-diène-11bêta-hexanoate d'éthyle,
- Acide 3-[(4,5-dihydro-1H-imidazol-2-yl)hydrazono]-17-oxo-.alpha.-[[(phénylméthoxy)carbonyl]amino]-estra-4,9-diène-11 bêta-hexanoïque,
- Acide 3-[(4,5-dihydro-1H-imidazol-2-yl)hydrazono]-17-hydroxy-.alpha.-[[(phénylméthoxy)carbonyl]amino]-estra-4,9-diène-11 bêta-hexanoïque,
- Acide 3-[(aminoiminométhyl)hydrazono]-17-oxoestra-4,9-dièn-11 bêta-pentanoique,
- Acide 3-[(1,4,5,6-tetrahydro-2-pyrimidinyl)hydrazono]-17-oxoestra-4,9-diène-11-bêta-hexanoïque,
- Acide 3-[(4,5-dihydro-1H-imidazol-2-yl)hydrazono]-17-oxoestra-4,9-diène-11 bêta-hexanoïque,
- Acide 4-chloro-3-[(4,5-dihydro-1H-imidazol-2-yl)hydrazono]-17-oxoestra-4,9-diène-11 bêta-hexanoïque,
- Acide 3-[(aminoiminométhyl)hydrazono]-4-chloro-17-oxoestra-4,9-diène-11 bêta-hexanoïque.
- Acide 3-[(aminoiminométhyl)hydrazono]-17-oxoestra-4,9-dièn-11-bêta-hexanoïque,
- Acide 6-[3-[(4,5-dihydro-1H-imidazol-2-yl)hydrazono]-17-méthylène-estra-4,9-diène-11 bêta-yl]-2-[[(phénylméthoxy)carbonyl]amino]-hexanoïque,
ainsi que leurs sels d'addition pharmaceutiquement acceptables.

6. Procédé de préparation des composés de formule (I) selon la revendication 1 comprenant les étapes suivantes :
a) On soumet un composé de formule (IIa) : 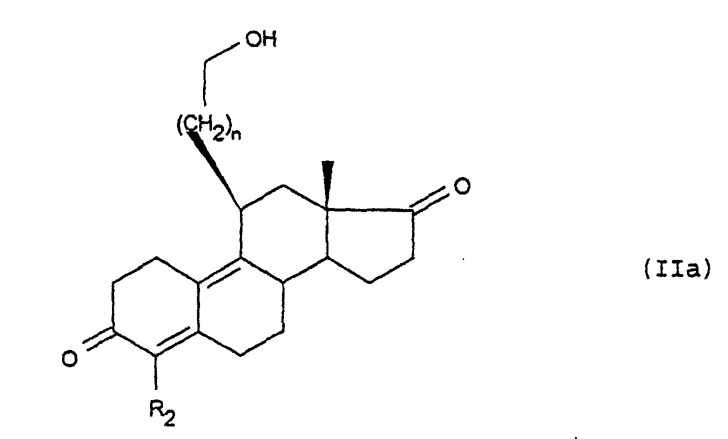 n étant un entier variant de 1 à 6, R₂ étant tel que défini à la revendication 1,
- d'abord à l'action d'un réactif d'halogénation ou d'un réactif d'activation de l'alcool,
- puis à l'action en présence de sodium, d'un composé de formule (F2) : 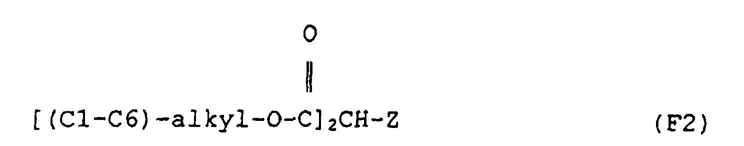
dans laquelle Z est tel que défini à la revendication 1 afin d'obtenir le composé de formule (III) : 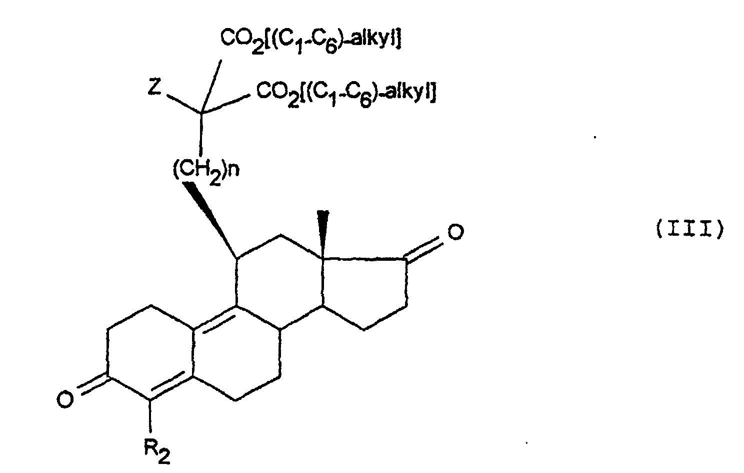
b) on soumet le composé de formule (III), à l'action d'un réactif de saponification puis de décarboxylation afin d'obtenir le composé de formule (IV) : 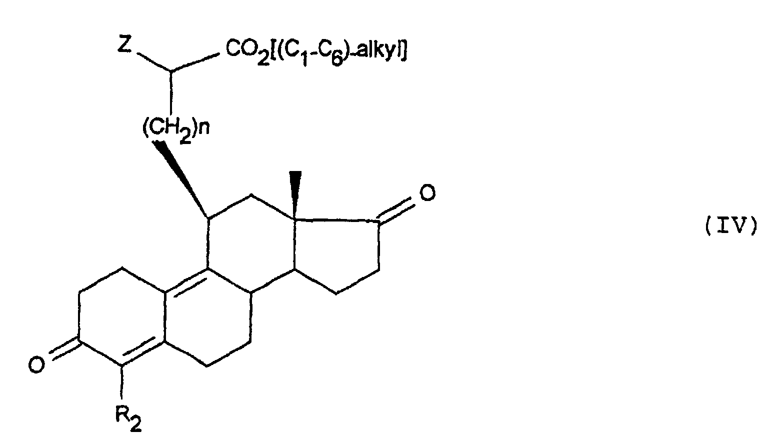
c) On soumet le composé de formule (IV) à l'action d'un composé de formule (F1) :
G-NH₂ F1)
dans laquelle G est tel que défini à la revendication 1 afin d'obtenir un composé de formule (I) qui si désiré ou si nécessaire, dans un ordre approprié subit l'une ou plusieurs des réactions suivantes :
- action d'un réactif d'halogénation en position 4 lorsque R₂ est un atome d'hydrogène,
- réduction en position 17 puis le cas échéant alkylation ou acylation,
- introduction du groupement méthylène en position 17,
- saponification,
- estérification ou amidification de la fonction acide,
- salification par un acide ou par une base.

7. Procédé selon la revendication 6 **caractérisé en ce que** les réactions d'halogénation en position 4, de réduction en position 17 suivi le cas échéant d'une alkylation ou d'une acylation et de l'introduction de groupement méthylène en position 17 peuvent s'effectuer au niveau des étapes a ou b, c'est-à-dire sur les composés de formule (IIa) ou sur les composés intermédiaires de formule (III) ou (IV).

8. Procédé de préparation des composés de formules (I) selon la revendication 1 avec Z représentant un atome d'hydrogène comprenant les étapes suivantes :
a) on soumet un composé de formule (IIb) : 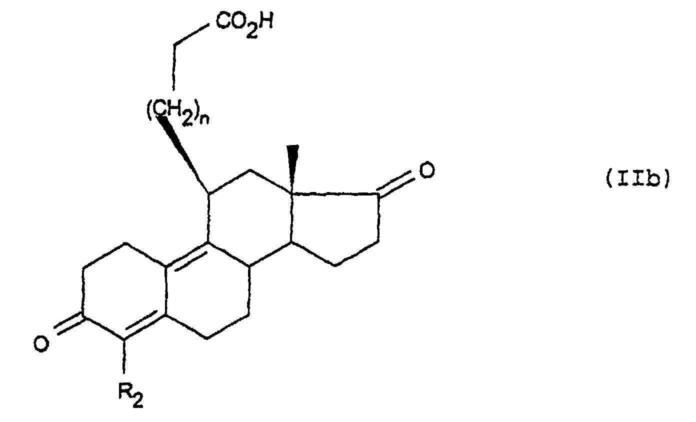
dans laquelle n est un entier variant de 1 à 6 et R₂ est un atome d'hydrogène,
- le cas échéant à l'action d'un réactif d'halogénation en position 4 afin d'obtenir le composé de formule (IIb) avec R₂ représentant un halogène,
- puis à l'action d'un composé de formule (F1) :
G-NH₂ (F1)
dans laquelle G est tel que défini à la revendication 1 afin d'obtenir un composé de formule (I) qui si désiré ou si nécessaire dans un ordre approprié subit l'une ou plusieurs des réactions suivantes :
- réduction en position 17 puis le cas échéant alkylation ou acylation,
- introduction du groupement méthylène en position 17,
- estérification ou amidification de la fonction acide,
- salification par un acide ou une base.

9. Procédé selon la revendication 8 **caractérisé en ce que** les réactions :
- de réduction en position 17, suivies le cas échéant d'une alkylation ou d'une acylation,
- d'introduction du groupement méthylène en position 17,
- ainsi que les réactions d'estérification, d'amidification ou de salification de la fonction acide peuvent s'effectuer préalablement sur le composé de formule (IIb).

10. A titre de médicaments, les composés de formule (I) ainsi que leurs sels d'addition pharmaceutiquement acceptables tels que définis à la revendication 1.

11. A titre de médicament les composés de formules (I) ainsi que leurs sels d'addition pharmaceutiquement acceptables tels que définis dans l'une quelconque des revendications 2 à 4.

12. A titre de médicament les composés de formules (I) ainsi que leurs sels d'addition pharmaceutiquement acceptables tels que définis à la revendication 5.

13. A titre de médicament ayant une activité antagoniste du récepteur de la vitronectine, un composé de formule (I) ainsi que leurs sels d'addition pharmaceutiquement acceptables tels que définis dans l'une quelconque des revendications 1 à 5.

14. A titre de médicament ayant une activité inhibitrice de la résorption osseuse ou pour le traitement ou la prévention de l'ostéoporose, un composé de formule (I) ainsi que leurs sels d'addition pharmaceutiquement acceptables tels que définis dans l'une quelconque des revendications 1 à 5.

15. A titre de médicament ayant une activité inhibitrice de la croissance tumorale ou des métastases cancéreuses, un composé de formule (I) ainsi que leurs sels d'addition pharmaceutiquement acceptables tels que définis dans l'une quelconque des revendications 1 à 5.

16. A titre de médicament ayant une activité anti-inflammatoire ou pour le traitement ou la prévention des désordres cardiovasculaires, la resténose, l'artériosclérose, les néphropathies ou rétinopathies, un composé de formule (I) ainsi que leurs sels d'addition pharmaceutiquement acceptables tels que définis dans l'une quelconque des revendications 1 à 5.

17. Les compositions pharmaceutiques renfermant un ou plusieurs médicaments selon l'une quelconque des revendications 10 à 16 et un ou plusieurs supports, véhicules, diluants ou adjuvants.

18. A titre de composés intermédiaires, les composés de formules (III) et (IV) ainsi que les composés de formules (IIa) et (IIb) dans lesquelles R₂ est un atome d'halogène tels que définis aux revendications 6 et 8.

## Patentansprüche

1. Verbindungen der allgemeinen Formel (I) 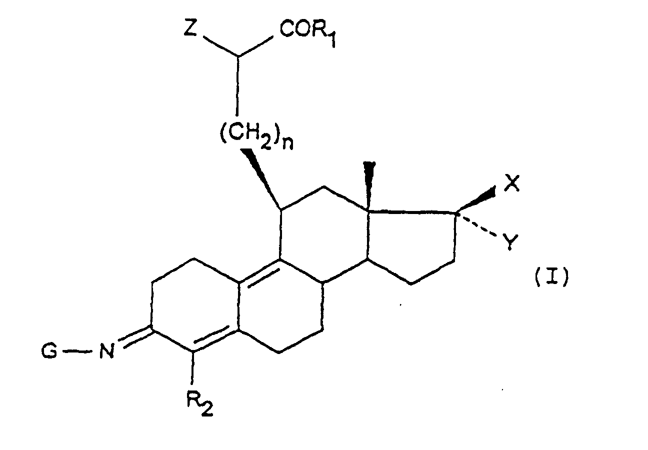 worin:
- X und Y entweder zusammen mit dem Kohlenstoff, der sie trägt, eine O=C- oder CH₂=C-Gruppierung bilden,
- oder X eine Hydroxy-, (C₁-C₆)-Alkyloxy- oder (C₁-C₆)-Alkylcarbonyloxy-Gruppe, worin der Alkyl-Rest nicht substituiert ist oder mit R₃ substituiert ist, darstellt; und Y ein Wasserstoffatom ist;
- R₁ eine Hydroxy-, (C₁-C₆)-Alkyloxy-, Amino-, (C₁-C₆)-Alkylamino- oder (C₂-C₁₂)-Dialkylamino-Gruppe,
worin der Alkyl-Rest nicht substituiert ist oder mit R₃ substituiert ist, darstellt;
- R₂ ein Wasserstoffatom oder ein Halogenatom darstellt;
- Z ein Wasserstoffatom, eine Gruppierung NHSO₂Ra, NHCO₂Ra, NHCORa, NHSO₂NHRa oder NHCONHRa darstellt;
- G entweder eine Gruppe 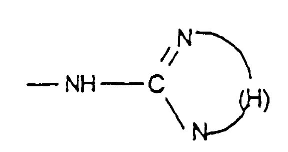 (Gruppe G1) darstellt, in der (H) mit dem Motiv -N=C-N-den Rest eines Heterocyclus bildet, der aus folgenden ausgewählt ist: 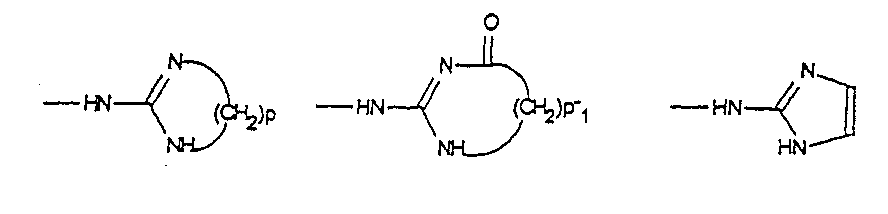 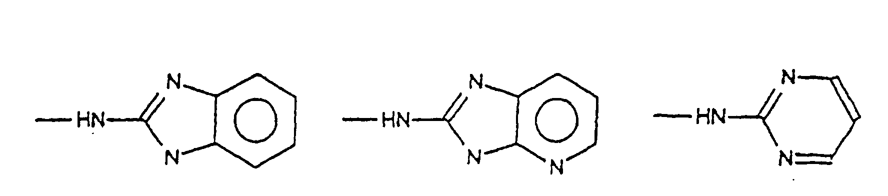 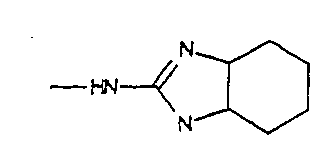 in denen die p eine ganze Zahl von 1 bis 4, vorzugsweise 2 oder 3, darstellen;
- oder eine Gruppe NRbRc (Gruppe G2) darstellt;
- oder einen aromatischen oder nicht-aromatischen, gesättigten oder ungesättigten Heterocyclus (Gruppe G3), der 1 bis 9 Kohlenstoffatome und 1 bis 5 Heteroatome, ausgewählt aus Sauerstoff-, Stickstoffund Schwefelatomen, umfaßt, darstellt;
- oder eine Gruppe NRb-C(=A)-NHRc (Gruppe G4),
worin A ein Schwefelatom, Sauerstoffatom oder NH ist, darstellt;
- oder eine Gruppe NRb-SO₂Rc (Gruppe G5) darstellt;
- Ra, Rb und Tc, die gleich oder unterschiedlich sind, ein Wasserstoffatom, eine Gruppe -(CH₂)ₘ-Alk oder -(CH₂)ₘ-Ar darstellen;
- der Ausdruck Alk eine Gruppe darstellt, die von einem linearen, verzweigten oder cyclischen, gesättigten oder ungesättigten, nicht-aromatischen Kohlenwasserstoff stammt, der 1 bis 12 Kohlenstoffatome umfaßt und mit R₃ substituiert ist oder nicht substituiert ist; Ar ein Phenyl darstellt, das mit R₃ substituiert ist oder nicht substituiert ist;
- n eine ganze Zahl von 1 bis 6 ist; m 0, 1, 2 oder 3 darstellt;
- der Substituent R₃ darstellt:
- Halogen, Oxo, Cyano, Nitro, Formyl, Carboxy, (C₁-C₆)-Alkyloxycarbonyl, Carboxamid,
- eine Alkyl-, Alkenyl- oder Alkinyl-Gruppe, die 1 bis 6 Kohlenstoffatome umfaßt und gegebenenfalls durch ein Halogenatom oder mehrere Halogenatome substituiert ist;
- eine Cycloalkyl-Gruppe, die 3 bis 12 Kohlenstoffatome umfaßt;
- eine Alkoxy- oder Alkylthio-Gruppe, die 1 bis 6 Kohlenstoffatome umfaßt;
- eine Amino-, Alkylamino-Gruppe, die 1 bis 6 Kohlenstoffatome umfaßt, oder eine Dialkylamino-Gruppe, die 2 bis 12 Kohlenstoffatome umfaßt, gegebenenfalls in oxidierter Form;
- eine Aminoalkyl-Gruppe, die 1 bis 6 Kohlenstoffatome umfaßt, oder eine Dialkylaminoalkyl-Gruppe, die 3 bis 8 Kohlenstoffatome umfaßt;
- eine Dialkylaminoalkyloxy-Gruppe, die 3 bis 18 Kohlenstoffatome umfaßt;
- eine Hydroxyl-Gruppe, gegebenenfalls acyliert, die 1 bis 12 Kohlenstoffatome umfaßt;
- eine Acyl-Gruppe, die 1 bis 12 Kohlenstoffatome umfaßt;
- eine Phenyl-, Phenylalkyl- oder Phenyloxy-Gruppe, wobei diese Gruppen gegebenenfalls selbst durch einen oder mehrere der oben genannten Substituenten substituiert sind;
wobei die Verbindungen der Formeln (I) in all ihren möglichen stereoisomeren Formen isoliert oder im Gemisch vorliegen, sowie ihre Ester und ihre Additionssalze mit Säuren und Basen, die pharmazeutisch annehmbar sind.

2. Verbindungen der Formel (I) nach Anspruch 1, in der R₁ ein Hydroxyl darstellt und G eine Gruppe -NH-C(=NH)-NHRc ist, sowie ihre pharmazeutisch annehmbaren Additionssalze.

3. Verbindungen der Formel (I) nach Anspruch 1, worin R₁ ein Hydroxyl darstellt und G unter den folgenden Heterocyclen ausgewählt ist: 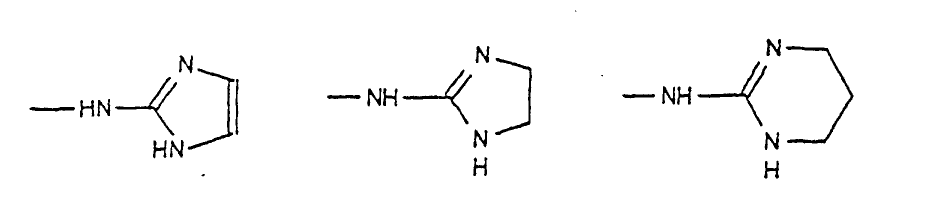 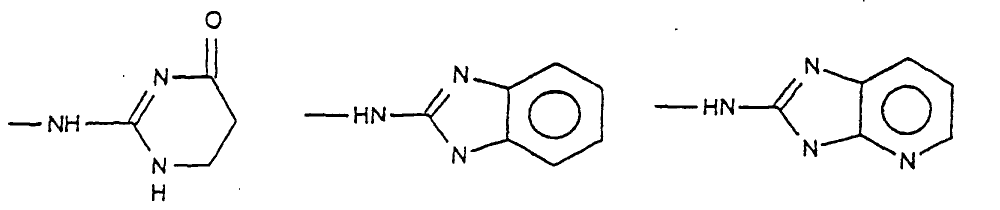 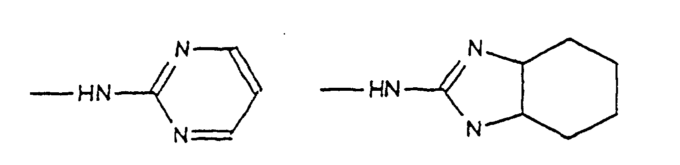 sowie ihre pharmazeutisch annehmbaren Additionssalze.

4. Verbindungen der Formel (I) nach Anspruch 1, worin Z ein Wasserstoffatom oder eine Gruppierung -NHCO₂CH₂Ph, -NHAc, -NHCO₂CH₂-Adamantyl ist und G eine Gruppierung
-NH-C(=NH) -NH₂, 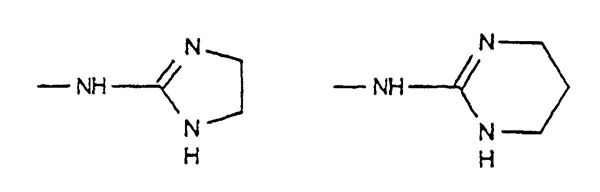
ist, sowie ihre pharmazeutisch annehmbaren Additionssalze.

5. Verbindungen der Formel (I) nach Anspruch 1 mit den folgenden Namen:
- 3-[(4,5-Dihydro-1H-imidazol-2-yl)hydrazono]-17-oxo-alpha-[[(phenylmethoxy)carbonyl]amino]-estra-4,9-dien-11-beta-hexansäureethylester;
- 3-[(4,5-Dihydro-1H-imidazol-2-yl)hydrazono]-17-oxo-alpha-[[(phenylmethoxy)carbonyl]amino]-estra-4,9-dien-11-beta-hexansäure;
- 3-[(4,5-Dihydro-1H-imidazol-2-yl)hydrazono]-17-hydroxy-alpha-[[(phenylmethoxy)carbonyl]amino]-estra-4,9-dien-11-beta-hexansäure;
- 3-[(Aminoiminomethyl)hydrazono]-17-oxoestra-4,9-dien-11-beta-pentansäure;
- 3-[(1,4,5,6-Tetrahydro-2-pyrimidinyl)-hydrazono]-17-oxo-estra-4,9-dien-11-beta-hexansäure;
- 3-[(4,5-Dihydro-1H-imidazol-2-yl)hydrazono]-17-oxo-estra-4,9-dien-11-beta-hexansäure;
- 4-Chlor-3-[(4,5-dihydro-1H-imidazol-2-yl)-hydrazono]-17-oxo-estra-4,9-dien-11-beta-hexansäure;
- 3-[(Aminoiminomethyl)hydrazono]-4-chlor-17-oxo-estra-4,9-dien-11-beta-hexansäure;
- 3-[(Aminoiminomethyl)hydrazono]-17-oxo-estra-4,9-dien-11-beta-hexansäure;
- 6-[3-[(4,5-Dihydro-1H-imidazol-2-yl)-hydrazono]-17-methylen-estra-4,9-dien-11-beta-yl]-2-[[phenylmethoxy)carbonyl]amino]hexansäure;
sowie ihre pharmazeutisch annehmbaren Additionssalze.

6. Verfahren zur Herstellung der Verbindungen der Formel (I) nach Anspruch 1, das die folgenden Schritte umfaßt:
a) man unterwirft eine Verbindung der Formel (IIa): 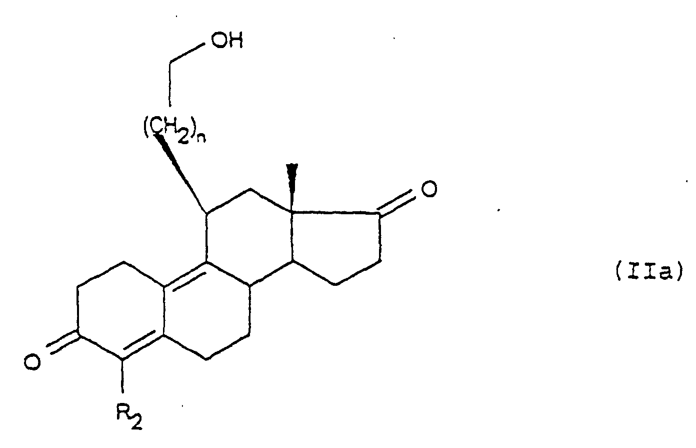 worin n eine ganze Zahl von 1 bis 6 ist, R₂ wie in Anspruch 1 definiert ist,
- zunächst der Einwirkung eines Halogenierungsreagenzes oder eines Aktivierungsreagenzes für den Alkohol,
- dann in Gegenwart von Natrium der Einwirkung einer Verbindung der Formel (F2): 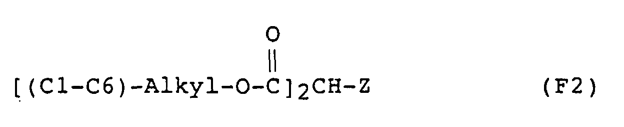
worin Z wie in Anspruch 1 definiert ist, um die Verbindung der Formel (III) zu erhalten: 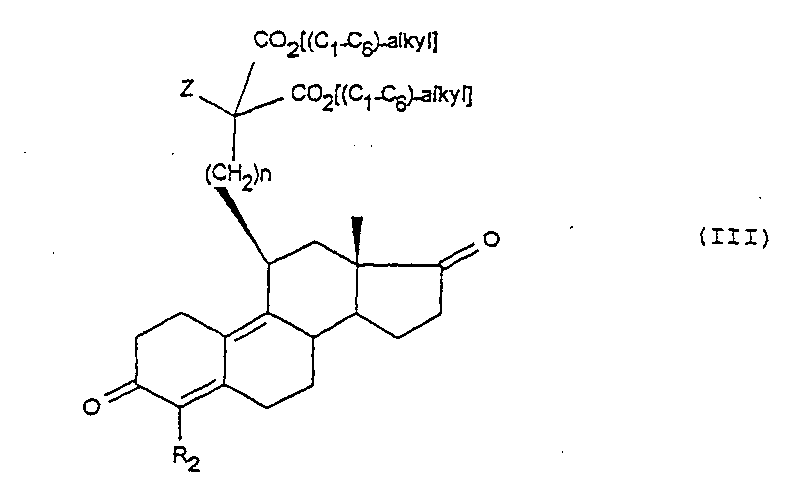
b) man unterwirft die Verbindung der Formel (III) der Wirkung eines Verseifungsreagenzes, dann einer Decarboxylierung, um die Verbindung der Formel (IV) zu erhalten: 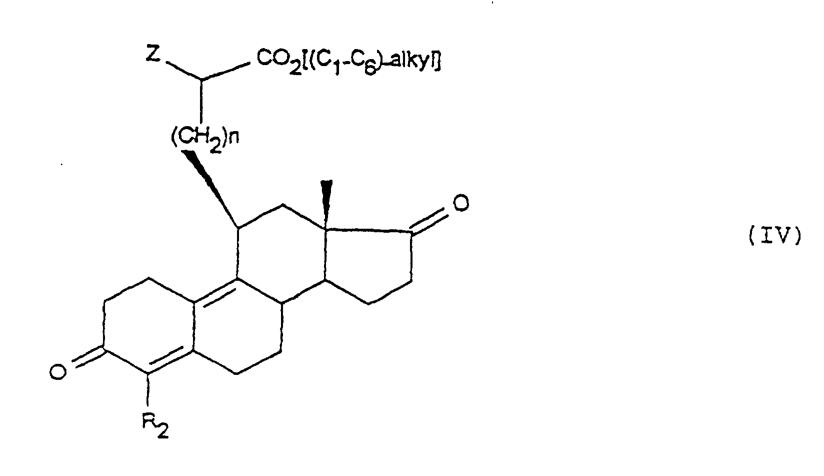
c) man unterwirft die Verbindung der Formel (IV) der Wirkung einer Verbindung der Formel (F1):
G-NH₂ (F1)
in der G wie in Anspruch 1 definiert ist, um eine Verbindung der Formel (I) zu erhalten, die, wenn gewünscht oder wenn notwendig, einer oder mehreren der folgenden Reaktionen in geeigneter Reihenfolge unterworfen wird:
- der Reaktion mit einem Halogenierungsreagens in Position 4, wenn R₂ ein Wasserstoffatom ist,
- einer Reduktion in Position 17, dann gegebenenfalls Alkylierung oder Acylierung,
- Einführung der Methylen-Gruppe in Position 17,
- Verseifung,
- Veresterung oder Amidierung der Säurefunktion,
- Salzbildung mit einer Säure oder einer Base.

7. Verfahren nach Anspruch 6, **dadurch gekennzeichnet, daß** die Halogenierungsreaktionen in Position 4, die Reduktion in Position 17, auf die gegebenenfalls eine Alkylierung oder Acylierung folgt, und die Einführung der Methylen-Gruppe in Position 17 auf dem Niveau der Schritte A oder B erfolgen können, d.h. mit den Verbindungen der Formel (IIa) oder den Zwischenverbindungen der Formel (III) oder (IV).

8. Verfahren zur Herstellung der Verbindungen der Formel (I) nach Anspruch 1, worin Z ein Wasserstoffatom darstellt, umfassend die folgenden Schritte:
a) man unterwirft eine Verbindung der Formel (IIb): 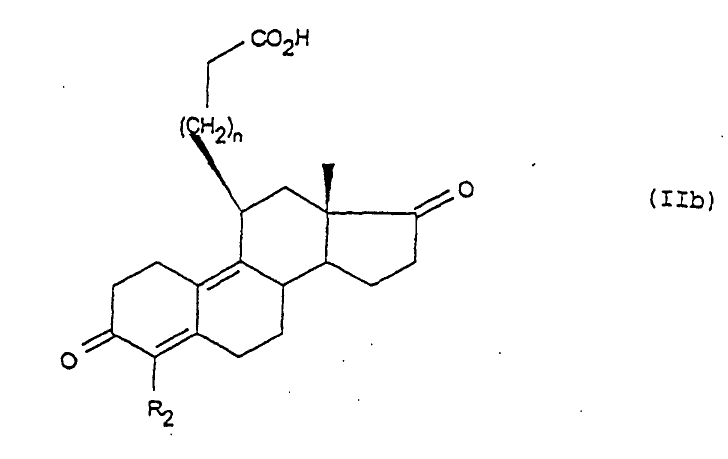 worin n eine ganze Zahl zwischen 1 und 6 ist und R₂ ein Wasserstoffatom ist;
- gegebenenfalls der Wirkung eines Halogenierungsreagenzes in Position 4, um die Verbindung der Formel (IIb) zu erhalten, wobei R₂ ein Halogen darstellt,
- unterwirft diese dann der Wirkung einer Verbindung der Formel (F1):
G-NH₂ (F1)
worin G wie in Anspruch 1 definiert ist, um eine Verbindung der Formel (I) zu erhalten, die, wenn gewünscht oder wenn notwendig, einer oder mehreren der folgenden Reaktionen unterworfen wird:
- Reduktion in Position 17, dann gegebenenfalls Alkylierung oder Acylierung,
- Einführung der Methylen-Gruppe in Position 17,
- Veresterung oder Amidierung der Säurefunktion,
- Salzbildung mit einer Säure oder oder einer Base.

9. Verfahren nach Anspruch 8, **dadurch gekennzeichnet, daß** die Reaktionen:
- Reduktion in Position 17, gegebenenfalls gefolgt von einer Alkylierung oder Acylierung,
- Einführung der Methylen-Gruppe in Position 17,
- sowie die Veresterungsreaktion, Amidierungsreaktion oder Salzbildungsreaktion der Säurefunktion vorher an der Verbindung der Formel (IIb) durchgeführt werden können.

10. Verbindungen der Formel (I) sowie ihre pharmazeutisch annehmbaren Additionssalze, wie sie in Anspruch 1 definiert sind, als Arzneimittel.

11. Verbindungen der Formel (I) sowie ihre pharmazeutisch annehmbaren Additionssalze, wie sie in einem der Ansprüche 2 bis 4 definiert sind, als Arzneimittel.

12. Verbindungen der Formel (I) sowie ihre pharmazeutisch annehmbaren Additionssalze, wie sie in Anspruch 5 definiert sind, als Arzneimittel.

13. Verbindung der Formel (I) sowie ihre pharmazeutisch annehmbaren Additionssalze, wie sie in einem der Ansprüche 1 bis 5 definiert sind, als Arzneimittel mit Antagonistaktivität für den Rezeptor von Vitronectin.

14. Verbindung der Formel (I) sowie ihre pharmazeutisch annehmbaren Additionssalze, wie sie in einem Ansprüche 1 bis 5 definiert sind, als Arzneimittel, das eine Hemmaktivität auf die Knochenresorption hat oder zur Behandlung oder Prävention von Osteoprose dient.

15. Verbindung der Formel (I) sowie ihre pharmazeutisch annehmbaren Additionssalze, wie sie in einem der Ansprüche 1 bis 5 definiert sind, als Arzneimittel, das Hemmaktivität auf das Tumorwachstum oder Krebsmetastasen hat.

16. Verbindung der Formel (I) sowie ihre pharmazeutisch annehmbaren Additionssalze, wie sie in einem der Ansprüche 1 bis 5 definiert sind, als Arzneimittel, das antiinflammatorische Wirkung hat oder zur Behandlung und Prävention kardiovaskulärer Krankheiten, Restenose, Arteriosklerose, Nephropathie oder Retinopathie dient.

17. Pharmazeutische Zusammensetzungen, die ein oder mehrere Arzneimittel nach einem der Ansprüche 10 bis 16 und einen oder mehrere Träger, Vehikel, Verdünnungsmittel oder Adjuvantien umfassen.

18. Verbindungen der Formeln (III) und (IV) sowie Verbindungen der Formeln (IIa) und (IIb), in denen R₂ ein Halogenatom ist, wie sie in den Ansprüchen 6 und 8 definiert sind, als Zwischenverbindungen.

## Claims

1. Compounds of general formula (I): 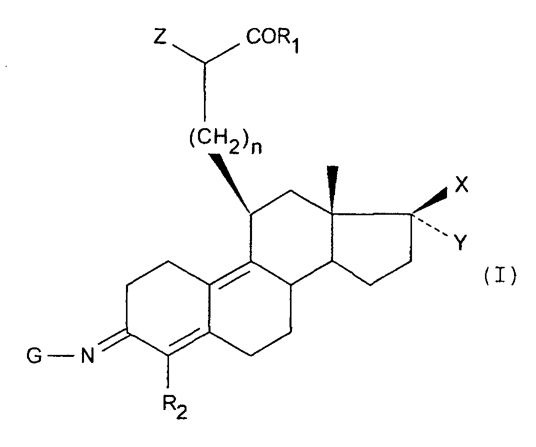 in which:
- either X and Y together with the carbon which carries them form a C=O or C=CH₂ group,
- or X represents a hydroxy, (C₁-C₆)-alkyloxy or (C₁₋C₆)-alkylcarbonyloxy radical the alkyl remainder being non substituted or substituted by R₃, and Y is a hydrogen atom;
- R₁ represents a hydroxy, (C₁-C₆)-alkyloxy, amino, (C₁-C₆)-alkylamino or (C₂-C₁₂)-dialkylamino radical, the alkyl remainder being non-substituted or substituted by R₃;
- R₂ represents a hydrogen or halogen atom;
- Z represents a hydrogen atom, an NHSO₂Ra, NHCO₂Ra, NHCORa, NHSO₂NHRa or NHCONHRa group;
- G represents:
- either an 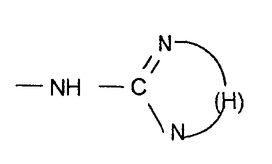 radical (G1 radical) in which (H) forms with the -N=C-N-unit the remainder of a heterocycle, chosen from 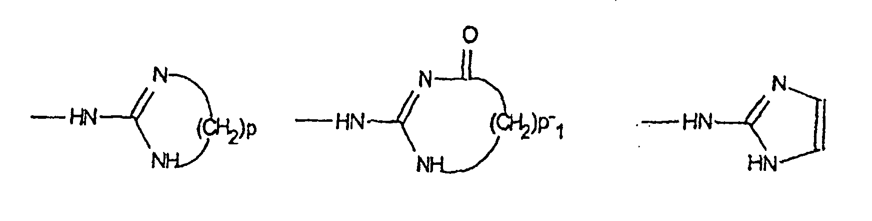 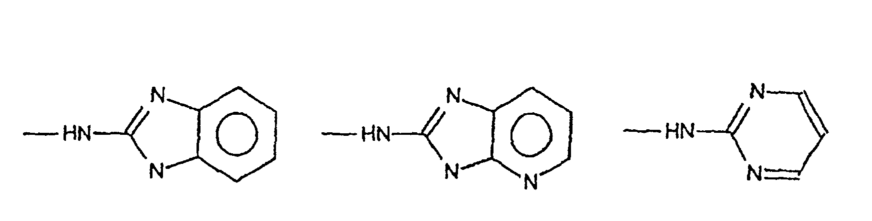 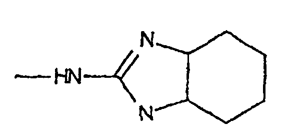 in which p represents an integer from 1 to 4 and preferably 2 or 3.
- or an NRbRc radical (G2 radical),
- or an aromatic or non aromatic, saturated or non saturated heterocycle (G3 radical), comprising from 1 to 9 carbon atoms and from 1 to 5 heteroatoms chosen from oxygen, nitrogen and sulphur atoms.
- or an NRb-C(=A)-NHRc radical (G4 radical) in which A is a sulphur, oxygen or NH atom,
- or an NRb-SO₂Rc radical (G5 radical),
- Ra, Rb and Rc, identical or different, represent a hydrogen atom, a -(CH₂)ₘ-Alk, or -(CH₂)ₘ-Ar radical,
- the term Alk representing a radical derived from a linear branched or cyclic, saturated or unsaturated, non aromatic hydrocarbon containing from 1 to 12 carbon atoms, substituted by R₃ or non substituted, Ar representing a phenyl substituted by R₃ or non substituted
- n is an integer ranging from 1 to 6, m represents 0,1,2 or 3,
- the substituent R₃ represents.
- halogen, oxo, cyano, nitro, formyl, carboxy, (C₁-C₆)-alkyloxycarbonyl, carboxamide,
- an alkyl, alkenyl or alkynyl radical containing from 1 to 6 carbon atoms optionally substituted by one or more halogen atoms,
- a cycloalkyl radical containing from 3 to 12 carbon atoms
- an alkoxy or alkylthio radical containing from 1 to 6 carbon atoms,
- an amino, alkylamino radical containing from 1 to 6 carbon atoms or dialkylamino radical containing from 2 to 12 carbon atoms optionally in oxidized form,
- an aminoalkyl radical containing from 1 to 6 carbon atoms or dialkylaminoalkyl radical containing from 3 to 8 carbon atoms,
- a dialkylaminoalkyloxy radical containing from 3 to 18 carbon atoms,
- an optionally acylated hydroxyl radical containing from 1 to 12 carbon atoms,
- an acyl radical containing from 1 to 12 carbon atoms,
- a phenyl, phenylalkyl or phenyloxy radical, these radicals being themselves optionally substituted by one or more substituents mentioned above,
said compounds of formulae (I) being in all their possible stereoisomer forms, isolated or in a mixture, as well as their esters and their addition salts with pharmaceutically acceptable acids and bases.

2. Compounds of formula (I) according to claim 1 in which R₁ represents a hydroxyl and G is an -NH-C(=NH)-NHRc radical, as well as their pharmaceutically acceptable addition salts.

3. Compounds of formula (I) according to claim 1 in which R₁ represents a hydroxyl and G is chosen from the following heterocycles: 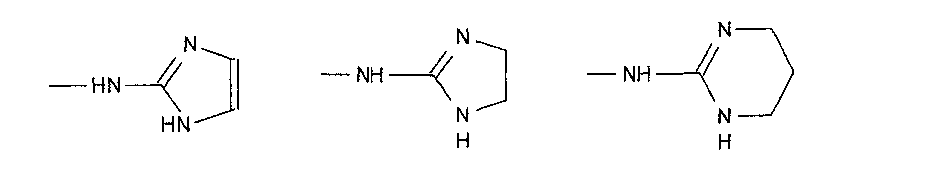 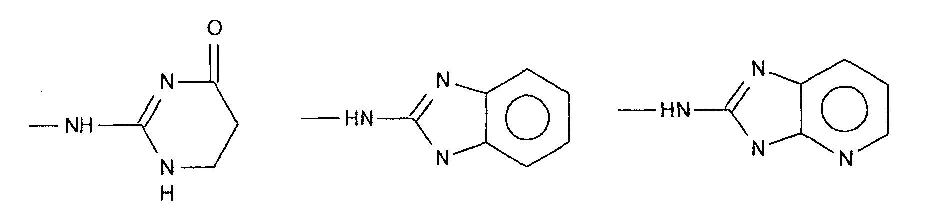 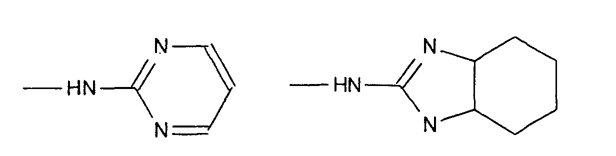 as well as their pharmaceutically acceptable addition salts.

4. Compounds of formula (I) according to claim 1 in which Z is a hydrogen atom or an -NHCO₂CH₂Ph, -NHAc, -NHCO₂CH₂-adamantyl group and G is an
-NH-C(=NH)-NH₂, 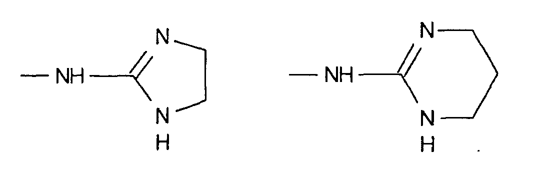
group as well as their pharmaceutically acceptable addition salts.

5. Compounds of formula (I) according to claim 1 the names of which follow:
- ethyl 3-[(4,5-dihydro-1H-imidazol-2-yl)hydrazono]-17-oxo-.alpha.-[[(phenylmethoxy)carbonyl]amino]-estra-4,9-dime-11 beta-hexanoate,
- 3-[(4,5-dihydro-1H-imidazol-2-yl)hydrazono]-17-oxo-.alpha.-[[(phenylmethoxy)carbonyl]amino]-estra-4,9-diene-11 beta-hexanoic acid,
- 3-[(4,5-dihydro-1H-imidazol-2-yl)hydrazono]-17-hydroxy-.alpha.-[[(phenylmethoxy)carbonyl]amino]-estra-4,9-diene-11 beta-hexanoic acid,
- 3-[(aminoiminomethyl)hydrazono]-17-oxoestra-4,9-dien-11 beta-pentanoic acid,
- 3-[(1,4,5,6-tetrahydro-2-pyrimidinyl)hydrazono]-17-oxoestra-4,9-diene-11-beta-hexanoic acid,
- 3-[(4,5-dihydro-1H-imidazol-2-yl)hydrazono]-17-oxo-estra-4,9-diene-11 beta-hexanoic acid,
- 4-chloro-3-[(4,5-dihydro-1H-imidazol-2-yl)hydrazono]-17-oxoestra-4,9-diene-11 beta-hexanoic acid,
- 3-[(aminoiminomethyl)hydrazono]-4-chloro-17-oxo-estra-4,9-diene-11 beta-hexanoic acid.
- 3-[(aminoiminomethyl)hydrazono]-17-oxoestra-4,9-dien-11-beta-hexanoic acid,
- 6-[3-[(4,5-dihydro-1H-imidazol-2-yl)hydrazono]-17-methylene-estra-4,9-diene-11 beta-yl]-2-[[(phenylmethoxy)carbonyl]amino]-hexanoic acid, as well as their pharmaceutically acceptable addition salts.

6. Process for the preparation of the compounds of formula (I) according to claim 1 comprising the following stages:
a) a compound of formula (IIa): 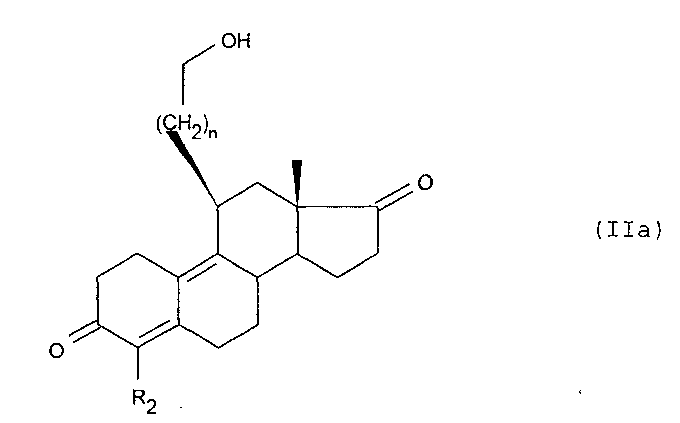 n being an integer ranging from 1 to 6, R₂ being such as defined in claim 1 is subjected,
- firstly to the action of a halogenation reagent or an activation reagent of alcohol,
- then in the presence of sodium, to the action of a compound of formula (F2): 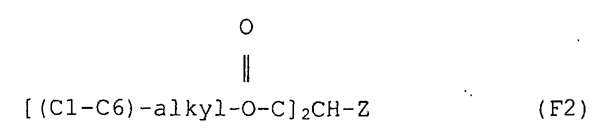 in which Z is as defined in claim 1 in order to obtain the compound of formula (III): 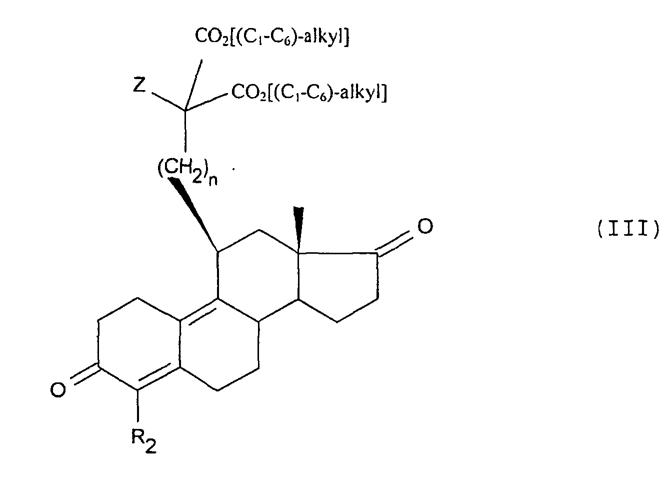
b) the compound of formula (III), is subjected to the action of a saponification reagent then a decarboxylation reagent in order to obtain the compound of formula (IV): 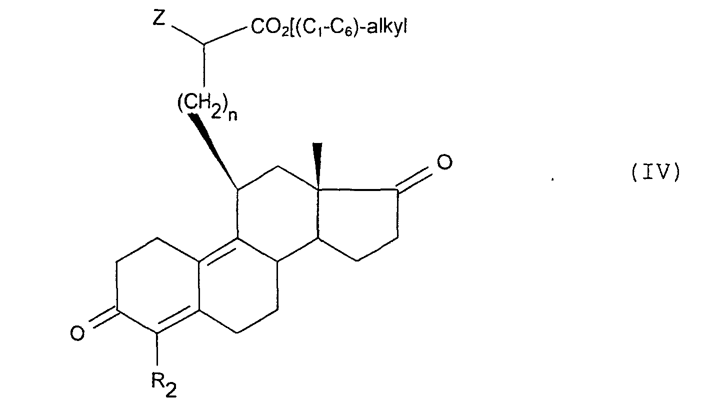
c) the compound of formula (IV) is subjected to the action of a compound of formula (F1):
G-NH₂ (F1)
in which G is as defined in claim 1 in order to obtain a compound of formula (I) which if desired or if necessary, is subjected in an appropriate order to one or more of the following reactions:
- action of a halogenation reagent in position 4 when R₂ is a hydrogen atom,
- reduction in position 17 then optionally alkylation or acylation,
- introduction of the methylene group in position 17,
- saponification,
- esterification or amidification of the acid function,
- salification by an acid or by a base.

7. Process according to claim 6 **characterized in that** the halogenation reactions in position 4, the reduction reactions in position 17 followed, if appropriate by an alkylation or acylation reaction and the introduction of a methylene group in position 17 can be carried out at the level of stages a or b, i.e. on the compounds of formula (IIa) or on the intermediate compounds of formula (III) or (IV).

8. Process for the preparation of the compounds of formulae (I) according to claim 1 with Z representing a hydrogen atom comprising the following stages:
a) a compound of formula (IIb): 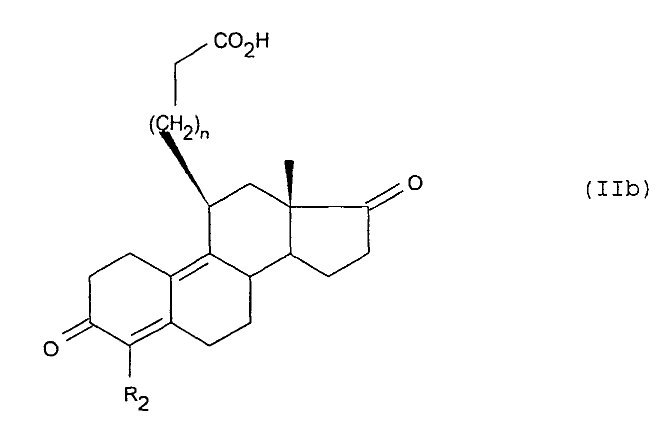 in which n is an integer ranging from 1 to 6 and R₂ is a hydrogen atom, is subjected
- if appropriate, to the action of a halogenation reagent in position 4 in order to obtain the compound of formula (IIb) with R₂ representing a halogen,
- then to the action of a compound of formula (F1):
G-NH₂ (F1)
in which G is as defined in claim 1 in order to obtain a compound of formula (I) which if desired or if necessary, is subjected in an appropriate order to one or more of the following reactions:
- reduction in position 17 then optionally alkylation or acylation,
- introduction of the methylene group in position 17,
- esterification or amidification of the acid function,
- salification by an acid or a base.

9. Process according to claim 8 **characterized in that** the following reactions:
- reduction in position 17, followed optionally by alkylation or acylation,
- introduction of the methylene group in position 17,
- as well as the esterification, amidification or salification reactions of the acid function can be carried out beforehand on the compound of formula (IIb).

10. As medicaments, the compounds of formula (I) as well as their pharmaceutically acceptable addition salts as defined in claim 1.

11. As a medicament the compounds of formulae (I) as well as their pharmaceutically acceptable addition salts as defined in any one of claims 2 to 4.

12. As a medicament the compounds of formulae (I) as well as their pharmaceutically acceptable addition salts as defined in claim 5.

13. As a medicament having an antagonist activity on the vitronectin receptor, a compound of formula (I) as well as their pharmaceutically acceptable addition salts as defined in any one of claims 1 to 5.

14. As a medicament having an inhibitory action on bone resorption or for the treatment or the prevention of osteoporosis, a compound of formula (I) as well as their pharmaceutically acceptable addition salts as defined in any one of claims 1 to 5.

15. As a medicament having an inhibitory action on tumourous growth or cancerous metastases, a compound of formula (I) as well as their pharmaceutically acceptable addition salts as defined in any one of claims 1 to 5.

16. As a medicament having an anti-inflammatory action or for the treatment or the prevention of cardiovascular disorders, the recurrence of stenosis, arteriosclerosis, nephropathies or retinopathies, a compound of formula (I) as well as their pharmaceutically acceptable addition salts as defined in any one of claims 1 to 5.

17. Pharmaceutical compositions containing one or more medicaments according to any one of claims 10 to 16 and one or more supports, vehicles, diluents or adjuvants.

18. As intermediate compounds, the compounds of formulae (III), (IV) and (V) as well as the compounds of formulae (IIa) and (IIb) in which R₂ is a halogen atom as defined in claims 6 and 8.
